(19) Europäisches Patentamt

European Patent Office

Office européen des brevets

(11) **EP 3 933 045 A1**

(12) **EUROPEAN PATENT APPLICATION**
published in accordance with Art. 153(4) EPC

(43) Date of publication:
**05.01.2022 Bulletin 2022/01**

(21) Application number: **19916567.1**

(22) Date of filing: **26.02.2019**

(51) Int Cl.:
**C12Q 1/02** (2006.01)

(86) International application number:
**PCT/JP2019/007245**

(87) International publication number:
**WO 2020/174568 (03.09.2020 Gazette 2020/36)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Hitachi High-Tech Corporation
Minato-ku
Tokyo 105-6409 (JP)**

(72) Inventors:
• **KANDA Katsuhiro**
  **Tokyo 105-6409 (JP)**
• **OOTOKO Kuniyo**
  **Tokyo 105-6409 (JP)**
• **KAI Akitsugu**
  **Tokyo 105-6409 (JP)**

(74) Representative: **MERH-IP Matias Erny Reichl
Hoffmann
Patentanwälte PartG mbB
Paul-Heyse-Strasse 29
80336 München (DE)**

(54) **METHOD OF DYNAMICS ANALYSIS FOR COMPOUND IN CELL**

(57)    Provided are a method and a technique for kinetic analysis of a compound such as a drug, using a cell. Specifically, disclosed is a method for kinetic analysis of a compound using a cell. The method includes a CE step of feeding a first buffer solution to a container containing a target compound and housing a cell, adjusting and keeping the internal temperature of the container to a first temperature, and adjusting the internal temperature of the container to a second temperature lower than the first temperature. Amounts S and S' of the compound in the first buffer solution are measured at a time interval t1 in the CE step, and based on the amounts S and S' and the time interval t1, an excretion rate of the compound excreted from a basal/basolateral face of the cell is determined.

FIG.1

**Description**

Technical Field

The present invention relates to in vitro (ex vivo) analysis and evaluation of an overview of kinetics of a compound (compound dynamics) including uptake, metabolism, and excretion of the compound.

Background Art

New drug development processes require clinical trials ("human clinical trials") of internally administering a candidate compound into the human body to prove its efficacy in consideration of the Pharmaceutical Affairs Law. However, human clinical trials and animal trials require immense development costs. Thus, demands have been made to prove the efficacy and toxicity of a drug candidate, particularly to evaluate the efficacy and toxicity using an in vitro (ex vivo) testing system employing human-derived cells, at a stage prior to such animal trials and human clinical trials.

A drug needs to be taken up by the liver and metabolized there into a metabolite or left as an original compound (a parent compound) without being metabolized, then excreted basolaterally, and recirculated in the bloodstream to the target organ in order to exhibit its efficacy in the body. Therefore, such an in vitro testing system that can measure the amount of a recirculating candidate pharmaceutical compound excreted basolaterally after administration and evaluate the efficacy could be a very useful pharmacokinetics evaluating system, where the efficacy is one of the most important indices in the new drug development. In addition, such a testing system would provide an overview of the pharmacokinetics including the amount excreted from cells into bile canaliculi (the amount of loss), which is the amount excreted into bile duct and then out of the body as feces, and the amount retained in the cells, and would thereby provide the distribution ratio of such fractions.

Patent Literature (PTL) 1 and PTL 2 disclose techniques of administering a drug to cultured cells and evaluating the amount of a fraction excreted to the bile canaliculi formed between cell-cell adhesion (the amount of loss). The techniques evaluate the amount of drug loss, where the administered drug undergoes biliary efflux and subsequent excretion outside the body as feces. The techniques are techniques specialized absolutely to the bile duct (bile) excretion evaluation.

The study group to which the inventors of the resent invention belong has developed methods and apparatuses for directly evaluating the amounts of components which are excreted into the blood vessels, to gain information of components having drug efficacy (PTL 3 and PTL 4). The methods and apparatuses evaluate not a portion of pharmacokinetics, but can quantitatively finely determine from which portion of cells the administered drug is excreted, and in which portion the administered drug accumulates, on the basis of the fractions of the administered drug, such as a fraction of basolateral (basal/basolateral) efflux, a fraction of apical efflux, and a fraction remained in cells. This provides an in vitro overview of pharmacokinetics and enables drug efficacy evaluation with higher accuracy.

Citation List

Patent Literature

PTL 1: PCT International Publication Number WO2005/118787
PTL 2: PCT International Publication Number WO94/12662
PTL 3: PCT International Publication Number WO2015/080106
PTL 4: PCT International Publication Number WO2016/166995

Summary of Invention

Technical Problem

The present invention has an object to provide a method and a technique (device) for kinetic analysis of a compound such as a drug using a cell, which kinetic analysis can be performed more rapidly and easily and can give more understandable and apprehendable results.

Solution to Problem

After intensive investigations to achieve the object, the inventors of the present invention have found that, in kinetic analysis of a compound using a cell, kinetics of the compound are more understandable and apprehendable by measuring excretion and/or uptake of the compound at two or more time points, and determining an excretion rate and/or uptake rate of the compound on the basis of the measurement results. The present invention has been made on the basis of these findings. The present invention includes, for example, embodiments as follows.

(1) A method for kinetic analysis of a compound using a cell.

The method includes a CE step. The CE step feeds a first buffer solution to a container containing a target compound and housing a cell, adjusts and keeps the internal temperature of the container to a first temperature, and adjusts the internal temperature of the container to a second temperature lower than the first temperature.

In the CE step, amounts S and S' of the compound in the first buffer solution are measured at a time interval t1.

On the basis of the amounts S and S' and the time interval t1, an excretion rate of the compound from a basal/basolateral face of the cell is determined.

(2) The method according to (1) for kinetic analysis of a compound,
in which the method includes a CE completion step, and an EA1 step after the CE completion step.

The CE step is conducted separately on a first container, a second container, and a third container each housing the cell and containing the compound, and the CE completion step collects the first buffer solution separately from the first container, the second container, and the third container.

The EA1 step feeds a second buffer solution containing a reagent for removing cell-cell adhesion to the first container, and adjusts and keeps the internal temperature of the first container to a third temperature.

In the EA1 step, amounts B1 and B1' of the compound in the second buffer solution are measured at a time interval t2.

On the basis of the amounts B1 and B1' and the time interval t2, an excretion rate of the compound excreted from the basal/basolateral face and an apical face of the cell is determined.

(3) The method according to (2) for kinetic analysis of a compound,
in which the method includes an EA2 step after the CE completion step. The EA2 step feeds a third buffer solution to the second container, and adjusts and keeps the internal temperature of the second container to the third temperature.

In the EA2 step, amounts B2 and B2' of the compound in the third buffer solution are measured at the time point t2.

On the basis of the amounts B2 and B2' and the time interval t2, an excretion rate of the compound excreted from the basal/basolateral face of the cell is determined.

(4) The method according to (3) for kinetic analysis of a compound,
in which an excretion rate of the compound excreted from the apical face of the cell is determined on the basis of the amounts B1 and B1', the amounts B2 and B2', and the time interval t2.

(5) The method according to (3) for kinetic analysis of a compound,
in which the method includes an EA3 step after the CE completion step. The EA3 step feeds a third buffer solution to the third container, and adjusts and keeps the internal temperature of the third container to a fourth temperature lower than the third temperature.

In the EA3 step, amounts B3 and B3' of the compound in the third buffer solution are measured at the time interval t2.

On the basis of the amounts B3 and B3' and the time interval t2, an excretion rate of the compound excreted by passive diffusion from the basal/basolateral face of the cell is determined.

(6) The method according to (5) for kinetic analysis of a compound,
in which an excretion rate of the compound via a transporter from the basal/basolateral face of the cell is determined on the basis of the amounts B2 and B2', the amounts B3 and B3', and the time interval t2.

(7) The method according to (1) for kinetic analysis of a compound,
in which the method includes a CE completion step, and an EA2 step after the CE completion step.

The CE step is conducted separately on a first container, a second container, and a third container each housing the cell and containing the compound, and the CE completion step collects the first buffer solution separately from the first container, the second container, and the third container.

The EA2 step feeds a third buffer solution to the second container, and adjusts and keeps the internal temperature of the second container to a third temperature.

In the EA2 step, amounts B2 and B2' of the compound in the third buffer solution are measured at a time interval t2.

On the basis of the amounts B2 and B2' and the time interval t2, an excretion rate of the compound excreted from the basal/basolateral face of the cell is determined.

(8) The method according to (1) for kinetic analysis of a compound,
in which the method includes a CE completion step, and an EA3 step after the CE completion step.

The CE step is conducted separately on a first container, a second container, and a third container each housing the cell and containing the compound, and the CE completion step collects the first buffer solution separately from the first container, the second container, and the third container.

The EA3 step feeds a third buffer solution to the third container, and adjusts and keeps the internal temperature of the third container to a fourth temperature lower than the third temperature.

In the EA3 step, amounts B3 and B3' of the compound in the third buffer solution are measured at a time interval t2.

On the basis of the amounts B3 and B3' and the time interval t2, an excretion rate of the compound excreted by passive diffusion from the basal/basolateral face of the cell is determined.

(9) A method for kinetic analysis of a compound using a cell,

the method includes a CE step, a CE completion step, and an EA1 step after the CE completion step.

The CE step feeds a first buffer solution to a first container, a second container, and a third container each containing a target compound and housing a cell, adjusts and keeps the internal temperatures of the containers to a first temperature, and adjusts the internal temperatures of the containers to a second temperature lower than the first temperature.

The CE completion step collects the first buffer solution separately from the first container, the second container, and the third container.

The EA1 step feeds a second buffer solution containing a reagent for removing cell-cell adhesion to the first container, and adjusts and keeps the internal temperature of the first container to a third temperature.

In the EA1 step, amounts B1 and B1' of the compound in the second buffer solution are measured at a time interval t2.

On the basis of the amounts B1 and B1' and the time interval t2, an excretion rate of the compound excreted from a basal/basolateral face and an apical face of the cell is determined.

(10) A method for kinetic analysis of a compound using a cell, the method includes a CE step, a CE completion step, and an EA2 step after the CE completion step.

The CE step feeds a first buffer solution separately to a first container, a second container, and a third container each containing a target compound and housing a cell, adjusts and keeps the internal temperatures of the containers to a first temperature, and adjusts the internal temperatures of the containers to a second temperature lower than the first temperature.

The CE completion step collects the first buffer solution separately from the first container, the second container, and the third container.

The EA2 step feeds a third buffer solution to the second container, and adjusts and keeps the internal temperature of the second container to a third temperature.

In the EA2 step, amounts B2 and B2' of the compound in the third buffer solution are measured at a time interval t2.

On the basis of the amounts B2 and B2' and the time interval t2, an excretion rate of the compound excreted from a basal/basolateral face of the cell is determined.

(11) A method for kinetic analysis of a compound using a cell,

the method includes a CE step, a CE completion step, and an EA3 step after the CE completion step.

The CE step feeds a first buffer solution separately to a first container, a second container, and a third container each containing a target compound and housing a cell, adjusts and keeps the internal temperatures of the containers to a first temperature, and adjusts the internal temperatures of the containers to a second temperature lower than the first temperature.

The CE completion step collects the first buffer solution separately from the first container, the second container, and the third container.

The EA3 step feeds a third buffer solution to the third container, and adjusts and keeps the internal temperature of the third container to a fourth temperature lower than the third temperature.

In the EA3 step, amounts B3 and B3' of the compound in the third buffer solution are measured at a time interval t2.

On the basis of the amounts B3 and B3' and the time interval t2, an excretion rate of the compound excreted by passive diffusion from a basal/basolateral face of the cell is determined.

(12) The method according to any one of (1) to (11) for kinetic analysis of a compound,

in which the method includes an uptake step before the CE step. The uptake step exposes the cell to a solution containing the compound in a container or containers to allow the cell to take up the compound.

In the uptake step, amounts A and A' of the compound in the solution are measured at a time interval t3.

On the basis of the amounts A and A' and the time interval t3, an uptake rate of the compound by the cell is determined.

(13) The method according to any one of (1) to (11) for kinetic analysis of a compound, in which the cell includes a hepatocyte.

Advantageous Effects of Invention

Application of the present invention enables more rapid and precise kinetic analysis of a compound in a cell, with more easy understanding and apprehension. In addition, the present invention enables quantification of fractions including a fraction of basolateral (basal/basolateral) efflux, a fraction of lumen-side (apical) efflux, and a fraction remained in the cell, of drug candidate compounds (a parent compound and its metabolites) via transporters and by diffusion. Determination of the totality and the distribution ratio of the administered drug candidate compounds to individual fractions enables evaluation of kinetics of the administered drug candidate compounds. This contributes to improved accuracy in in-vitro screening of drug candidates exhibiting a drug efficacy, from among an enormous number of drug candidate compounds.

Brief Description of Drawings

Fig. 1 is a graph illustrating the concept of kinetic analysis of a compound;
Fig. 2 illustrates a sample preparation flow;
Fig. 3 illustrates fractions quantified in Steps 4, 5, and 6 and image view of compound distribution;
Fig. 4 illustrates definitions of fractions and results of fluorescence reagent CDF measurement;
Fig. 5 is a graph illustrating a distribution ratio of fluorescence reagent CDF;
Fig. 6 is a graph illustrating a distribution ratio of fluorescence reagent Rhodamine 123;
Fig. 7 illustrates a culture plate;
Fig. 8 illustrates a configuration of an automatic measuring apparatus;
Fig. 9 is a top view of a sample preparation unit;
Fig. 10 is a front view of the sample preparation unit;
Fig. 11(a) is a flow chart of operations of the automatic measuring apparatus; and
Fig. 11(b) is a flow chart of operations of the automatic measuring apparatus.

Description of Embodiments

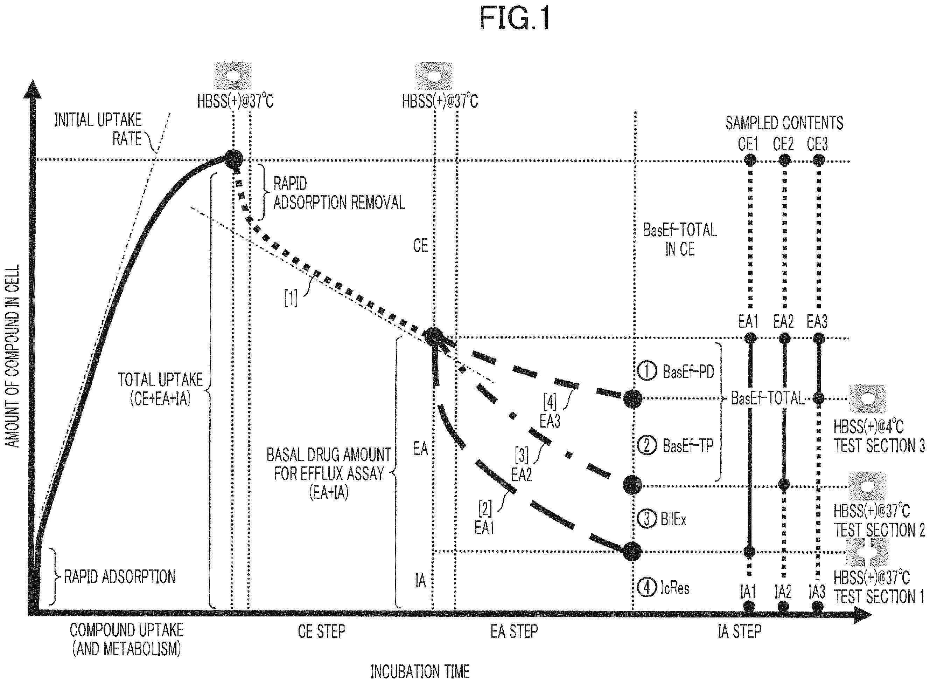

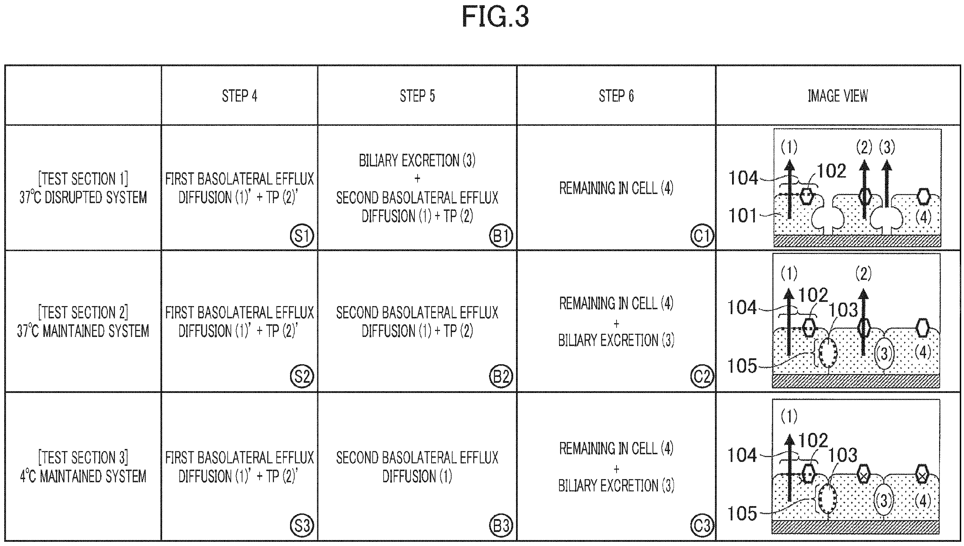

The present invention relates to improvements in a method for (in vitro) kinetic analysis of a compound in cells. A general outline of the method for kinetic analysis of a compound, which some of the inventors have previously developed (PTL 3 and PTL 4), is as follows. Specifically, the method uses three Test Sections 1 to 3 as illustrated in Fig. 3.
In Test Section 1, a target compound is excreted from cells to a buffer solution (such as Hanks' (-) solution), as a total of a biliary efflux (3) and a fraction of second basolateral efflux (passive diffusion (1) and efflux via a transporter (TP) (2)).
In Test Section 2, bile ducts are not disrupted, unlike Test Section 1, and the compound is excreted only by the fraction of second basolateral efflux (passive diffusion (1) and transporter (2)).
Test Section 3 has a temperature condition lower than that in Test Sections 1 and 2, and thereby the excretion of the compound via the transporter (1) is restrained and only the compound excreted by passive diffusion (2) is excreted in the fraction of second basolateral efflux.
The kinetic analysis method quantitatively analyzes the amount of the compound collected in at least one of Test Sections 1 to 3 and thereby enables determination of, for example, the amount of the compound excreted to the bile ducts (biliary efflux (3)) and the amount of the compound in the fraction of second basolateral efflux, each by subtracting the qualified amount of the compound collected in Test Section 2 from the quantified amount of the compound collected in Test Section 1. In addition, the method also enables determination (quantification) of the compound in basolateral efflux via the transporter, on the basis of comparison between Test Section 2 and Test Section 3. For each compound, a specific transporter is present for moving between inside and outside of the cell. Therefore, the ability to exclude the amount of excretion by passive diffusion and to quantify the amount of excretion via the transporter means the ability to quantify the amount of excretion specific to the compound.
The method according to the present invention measures excretion of the compound from the cell at at least two points in time, and determines an excretion rate of the compound, to perform the kinetic analysis method more rapidly and easily, with visual understanding of results (Fig. 1). Uptake of the compound to the cell may be measured at at least two points in time to determine an uptake rate of the compound (Fig. 1). Determination of the excretion rate (and optionally, the uptake rate) of the compound enables analysis of kinetics of the compound typically before the completion of cell culture step in Test Sections 1 to 3. The method also enables more precise analysis of variations (changes) and prediction of subsequent kinetics of the compound, as compared with measurements of actual values alone. In addition, the method enables visual understanding of kinetics of the compound by preparing a graph as illustrated in Fig. 1.

In the method, the term "cell" refers to any cell for which kinetics, such as uptake and/or excretion, of a compound are to be evaluated, and is not limited. The cell can be derived from any living body without limitation. Examples of the cell usable herein include cells derived from any living bodies such as vertebrates (e.g., mammals, birds, reptiles, fishes, and amphibians), invertebrates (e.g., insects, roundworms, and shellfishes), protozoans, plants, fungi, and bacteria. The cell is preferably a mammal cell, and particularly preferably a human cell. The cell is not limited also in its type, and is preferably a cell suited for the evaluation of biliary efflux or blood (basolateral) excretion, such as a hepatocyte. The cell is available by a technique that is easily understood by a person skilled in the art. For example, the cell may be prepared by a preparation technique commonly used in the field, or is available typically as a commercially available cell or a deposited cell (such as a cell line).

In the method, the compound to be evaluated is any compound whose kinetics in the cell, such as uptake to the cell and/excretion from the cell, is to be evaluated, and is not limited. Specifically, Non-limiting examples of the compound include naturally occurring compounds such as amino acids, peptides, oligopeptides, polypeptides, proteins, nucleic acids, lipids, carbohydrates (such as sugars), steroids, glycopeptides, glycoproteins, and proteoglycans; synthesized analogs or derivatives of naturally occurring compounds, such as peptide mimics, and nucleic acid molecules (e.g., aptamers, antisense nucleic acids, and double-strand RNAs (RNAis)); compounds that do not naturally occur, such as low-molecular weight organic compounds (such as an inorganic and organic compound library, or a combinatorial library); and mixtures of them. The compound may be a single substance, or a composite including substances, or, for example, a foodstuff or diet.

In one embodiment, the present disclosure provides a method for kinetic analysis of a compound using a cell.

The method includes a CE step. The CE step feeds a first buffer solution containing a target compound and housing a cell, adjusts and keeps the internal temperature of the container to a first temperature, and adjusts the internal temperature of the container to a second temperature lower than the first temperature.

In the CE step, amounts S and S' of the compound in the first buffer solution are measured at a time interval t1.

On the basis of the amounts S and S' and the time interval t1, an excretion rate of the compound excreted from a basal/basolateral face of the cell is determined.

The method determines a time-dependent change (a slope in the graph) [1] in the CE step in Fig. 1. For S1, S2, and S3 shown in Fig. 3, $S1 \approx S2 \approx S3$. Thus, the testing section in which measurement of S and S' (sampling of the compound in the first buffer solution) operates may be any one of Test Sections 1 to 3 in Fig. 3.

As used herein, the term "CE step" refers to a conditioning efflux step and is the step of conditioning cells (corresponding to Step 4 in the after-mentioned embodiments). The CE step operates after cell washing at a low temperature (e.g., 4°C), which is an upstream step (corresponding to Step 3 in the embodiments). The CE step cultures the cells at a culture temperature (e.g., 37°C) for a predetermined time, cools the culture container, and collects a supernatant fraction. The CE step can remove a portion of the compound adsorbed by the cells, which has not yet been removed completely in the upstream step and can restrain the influence of the portion on the excretion evaluation data obtained in downstream steps. If the CE step is omitted, the adsorbed portion of the compound (i.e., noise) is to be included in a fraction obtained in a downstream step (corresponding to Step 5 in the embodiments) subsequent to the CE step. In addition, the CE step can stabilize excretion evaluation by the working of excretion deceleration in steps after the CE step, by which the amount of the compound in the cells decreases. For example, the configuration can reduce the influence of a delicate temporal difference in simultaneous sampling of multiple samples. On the other hand, typically for a compound whose adsorption is negligible, the CE step may be shortened, or omitted, (or minimized).

Comparison between quantified values of the supernatant fraction in the CE step enables checking of the states of cells and liquid handling among samples (e.g., among wells) during operations from the cell culture to the CE step. This enables, for example, grasping and removal of a singular value. Specifically, the configuration enables checking of the states of the cells themselves during operations from the cell culture to the CE step, and checking whether the liquid handling during operations up to the CE step are equivalent among the samples.

The CE step, if performed for an excessively long time, decreases the concentration of the compound in the cells for downstream steps, and this tends to give fluctuations or variations in the analysis data. In contrast, the CE step, if performed for an excessively short time, may fail to sufficiently remove the compound adsorbed by the cells, and this may affect the analysis data in the downstream steps. To eliminate or minimize these, the time period of the CE step is appropriately set according typically to the cell type and the target compound type.

Specifically, the CE step feeds a first buffer solution (such as Hanks' solution) to a container including a compound to be evaluated and containing cells, adjusts and keeps the internal temperature of the container to a first temperature (e.g., 37°C), and adjusts the internal temperature of the container to a second temperature (e.g., 4°C) lower than the first temperature.

The container to contain or house the cells can be any container that is known in the field and used for cell culture, and can be selected appropriately according typically to the cell type. When two or more containers are used, the containers may be separated containers or a container including wells, such as a multi-titer plate.

The container contains (houses) the cells and the compound to be evaluated (target compound). The number of cells and the amount (concentration) of the compound can also be set appropriately.

For allowing the cells to maintain their functions, the first buffer solution is based typically on a balanced salt solution or physiological saline solution, includes inorganic salts, is adjusted under isotonic conditions, and has buffer capacity. The first buffer solution can be selected appropriately according to the cell type. Non-limiting examples of the first buffer solution usable herein include Hanks' balanced salt solution, Earle balanced salt solution, Dulbecco phosphate-buffer physiological saline solution, phosphate-buffer physiological saline solution, and Puck's balanced salt solution. The amount of the first buffer solution to be fed to the container is set appropriately according typically to the cell type, the culture (incubation) time, and the planed number of sampling.

After feeding the first buffer solution, the internal temperature of the container is adjusted and kept to the first temperature. The first temperature is a most suited temperature for the cell culture and can be selected appropriately according to the cell type. In general, the first temperature is for example about 37°C, which is the body temperature assuming an in vivo environment. Next, the internal temperature of the container is adjusted to a second temperature lower than the first temperature. The second temperature is such a temperature as to restrain the cells, which have take up the compound, from excreting the compound to the outside and can be selected appropriately according to the cell type. In general, the second temperature is typically a temperature under cooling (ice cooling) of about 4°C.

In the CE step, amounts S and S' of the compound in the first buffer solution are measured at a time interval t1. Specifically, sampling is performed at least two times at the time interval t1, and the amounts of the compound in the first buffer solution are measured. To more precisely determine a time-dependent change, namely, velocity or rate, sampling is preferably performed two or more times. However, in some cases, first sampling may be performed at the start of the step, for the sake of convenience.

Multiple sampling operations may be performed by any technique. For example, the method can employ the technique of providing different wells for different points in time (hereinafter also referred to as "time point(s)") of sampling. When sampling is performed at two time points, it is enough to provide at least two wells under identical conditions. Alternatively, sampling can be performed multiple times by sampling the buffer solution from the same well multiple times. This technique enables analysis of the excretion rate of the compound in the same well, namely, from the same cell. The technique of sampling the buffer solution from the same well can be further roughly classified as a supply technique and a non-supply technique. In the supply technique, the buffer solution in an amount the same as with the sampling amount is supplied to the well after one sampling. However, the buffer solution in the well after supply has a low concentration of the compound as compared with the buffer solution before supply. In the non-supply technique, the buffer solution is not supplied to the well after one sampling. However, the buffer solution in the well after sampling decreases in volume, and the compound in the well after a predetermined time course increases in concentration as compared with the buffer solution in a well from which the solution is not sampled, provided that the compound discharges from the cells at a constant excretion rate. As described above, the technique of sampling the buffer solution from the same well may affect the concentration of the compound in the buffer solution in the well. Preferably, an appropriate technique is selected according to the properties of the cells and the target compound, or two or more techniques may be trialed.

The time points are preferably set at points in a region where the concentration curve in the step (CE step) forms a straight line (where the uptake rate or excretion rate of the compound to or from the cells stands constant) (for example, straight line [1] illustrated in Fig. 1). However, in many cases, it is unknown that at which point in time the concentration curve forms a straight line. The uptake rate and excretion rate of the compound may vary vigorously immediately after the start of, and immediately after the completion of, the step. The time points are therefore preferably set at the midpoint of the step. Although optimal time points may vary depending on the target compound type and the cell type, the CE step may employ at least two, and preferably two or more time points at a time interval of typically several seconds to several tens of minutes. Setting of the time points appropriately as above enables appropriate analysis of the kinetics (velocity or rate) of the compound in the CE step. In some cases, however, first sampling may be performed at the start of the step for the sake of convenience.

For example in one embodiment, two measurement operations are performed at a time interval t1 during a constant state of the internal temperature of the container (under the same condition), such as at the first temperature.

The amount of the compound in the first buffer (first buffer solution) can be measured using a technique or reagent which a person skilled in the art commonly uses, according to the target compound type. The measurement operations at time points are preferably performed using the same technique or reagent.

As used herein, the term "measurement of a compound" means measurement of the amount or concentration of the compound or a metabolite thereof in the buffer preferably semi-quantitatively or quantitatively. The amount may be an absolute amount or a relative amount. The measurement can be performed directly or indirectly. The direct measurement includes measurement of the amount or concentration of the compound on the basis of a signal that directly correlates to the number of molecules of the compound. The indirect measurement is measurement of a signal derived from a secondary component (namely, a component other than the compound) such as a ligand, a label, or an enzymatic reaction product.

When kinetics of not the compound itself, but a metabolite of the compound is to be analyzed, a determination or calculation according to an analysis formula may be performed with the denominator and the numerator being the number of molecules per protein or per cell. The measured concentrations are each converted to a number of molecules. In contrast, when the compound itself is to be analyzed, the determination can also be performed using the concentration instead of the number of molecules. When the concentration is used, the determination operates in consideration of the sampling rate (sampling ratio).

Analysis of the time-dependent change (slope [1] in Fig. 1) in the CE step by the above procedure can determine an excretion rate of the compound excreted from the basal/basolateral face of the cell, namely, a total blood excretion rate (by passive diffusion plus via transporter). Specifically, the excretion rate of the compound from the basal/basolateral face of the cell is determined on the basis of the compound amounts S and S' measured at the time interval t1 according to the formula: $(S'-S)/t1$

As described above, chronological analysis in the CE step enables measurement of the initial rate of total excretion (totality of amounts of blood excretion (basolateral excretion) = active excretion plus passive excretion).

In another embodiment, the present disclosure provides a method for kinetic analysis of a compound using a cell. The method includes a CE completion step, and an EA1 step after the CE completion step.

The CE step is performed separately on a first container, a second container, and a third container each housing the cell and containing the compound; and the CE completion step collects the first buffer solution separately or independently from the first container, the second container, and the third container.

The EA1 step, after the CE completion step, feeds a second buffer solution containing a reagent for removing cell-cell adhesion to the first container, and adjusts and keeps the internal temperature of the first container to a third temperature.

In the EA1 step, amounts B1 and B1' of the compound in the second buffer solution are measured at a time interval t2.

On the basis of the amounts B1 and B1' and the time interval t2, an excretion rate of the compound excreted from the basal/basolateral face and an apical face of the cell is determined.

The method determines a time-dependent change (slope of the graph) [2] in the EA step in Fig. 1. The test section in which B1 and B1' are measured (the compound in the second buffer solution is sampled) is Test Section 1 in Fig. 3.

Initially, the CE completion step operates. The CE completion step performs the CE step separately on a first container, a second container, and a third container each housing the cell and containing the compound, and collects the first buffer solution separately from the first container, the second container, and the third container.

Subsequently, the EA step operates. Herein the term "EA step" means an efflux assay step, and is a step for identifying and evaluating the amounts of the compound excreted from the cell into the bile and blood (via transporter and passive diffusion) under the same conditions (step and sample). The EA step corresponds to Step 5 in the embodiments. In the method, the EA step is classified according to the test sections into an EA1 step, an EA2 step, and an EA3 step corresponding respectively to Test Sections 1 to 3.

Specifically, the EA1 step operates after the CE completion step, and feeds the second buffer solution containing the reagent removing cell-cell adhesion to the first container, and adjusts and keeps the internal temperature of the first container to the third temperature.

Removal (disengage) of cell-cell adhesion of the cells using the reagent for removing cell-cell adhesion allows all portions of the compound other than the compound remaining in the cells to be excreted or released (Test Section 1, 37°C disrupted system in Fig. 3). The reagent for removing cell-cell adhesion can be selected from reagents known in the technical field, and non-limiting examples thereof usable herein include chelators (such as glycol ether diaminetetraacetic acid (EGTA)). EGTA has chelating action of restraining the activities of $Ca^{2+}$ and $Mg^{2+}$, which are involved in adhesion of cell-cell adhesion molecules, and is well known as a reagent for removing cell-cell adhesion.

The second buffer solution (such as Hanks' (-) solution) containing the reagent removing cell-cell adhesion is fed to the first container, and the internal temperature of the first container is adjusted to the third temperature. The third temperature is a temperature most suited for incubation of the cells and can be appropriately selected according to the cell type. In general, the third temperature is typically about 37°C, which is a body temperature assuming an in vivo environment.

In the EA1 step, amounts B1 and B1' of the compound in the second buffer solution are measured at a time interval t2. Specifically, the amounts of the compound in the second buffer solution are measured by sampling at least two times at a time interval t2. Although optimal time points of sampling vary depending on the target compound type and the cell type, the EA1 step may employ at least two, and preferably two or more time points at a time interval of typically several seconds to several tens of minutes. In some cases, however, first sampling may be performed at the start of the step for the sake of convenience.

Multiple operations of sampling and compound amount measurement are as described above. In Test Section 1 (37°C disrupted system in Fig. 3), cell-cell adhesion is disrupted (and components present in the bile duct discharge to the outside with time).

In Test Section 1, the compound is excreted from the cells into the buffer solution (such as Hanks' (-) solution) in an amount equal to the sum of biliary efflux (3) and the fraction of second basolateral efflux (that is, excretion by passive diffusion (1) and via transporter (TP) (2)) (Fig. 3). Accordingly, analysis of a time-dependent change (slope [2] in Fig. 1) in the EA1 step by the above procedure enables determination of the excretion rate of the compound excreted from the basal/basolateral face and the apical face of the cells. Specifically, on the basis of the compound amounts B1 and B1' measured at the time interval t2, the excretion rate of the compound excreted from the basal/basolateral face and the apical face of the cells is determined according to the formula: (B1-B1')/t2.

In another embodiment, the present disclosure provides a method for kinetic analysis of a compound using a cell. The method includes the CE completion step, and an EA2 step after the CE completion step. The EA2 step feeds a third buffer solution to the second container, and adjusts and keeps the internal temperature of the second container to a third temperature.

In the EA2 step, amounts B2 and B2' of the compound in the third buffer solution are measured at a time interval t2.

On the basis of the amounts B2 and B2' and the time interval t2, an excretion rate of the compound excreted from the basal/basolateral face of the cells is determined.

This method determines a time-dependent change (slope of the graph) [3] in the EA step in Fig. 1. The test section for which B2 and B2' are measured (the compound in the third buffer solution is sampled) is Test Section 2 in Fig. 3.

Initially, the CE completion step operates as described above. Next, the EA2 step, which operates after the CE completion step, feeds the third buffer solution to the second container, and adjusts and keeps the internal temperature of the second container to the third temperature.

The third buffer solution is a buffer solution suited for cell culture, and a non-limiting example thereof usable herein is the first buffer solution (Hanks' solution). The third temperature is as in the EA1 step.

In the EA2 step, amounts B2 and B2' of the compound in the third buffer solution are measured at the time interval t2. Specifically, the amounts of the compound in the third buffer solution are measured by sampling the buffer at least two times at the time interval t2. Although optimal time points of sampling vary depending on the target compound type and the cell type, the EA2 step may employ at least two, and preferably two or more time points at a time interval of typically several seconds to several tens of minutes. Multiple operations of sampling and compound amount measurement are as described above. In some cases, however, first sampling may be performed at the start of the step for the sake of convenience.

In Test Section 2, the compound is excreted only as the fraction of second basolateral efflux (passive diffusion (1) and transporter (2)) (Fig. 3). Accordingly, analysis of the time-dependent change (slope [3] in Fig. 1) in the EA2 step by the procedure enables determination of the excretion rate of the compound excreted from the basal/basolateral face of the cells. Specifically, the excretion rate of the compound from the basal/basolateral face of the cells is determined from the compound amounts B2 and B2' measured at the time interval t2 according to Formula: (B2'-B2)/t2.

In yet another embodiment, the present disclosure provides a method for kinetic analysis of a compound using a cell.

The method includes the CE completion step, and after this step, an EA3 step. The EA3 step feeds a third buffer solution to the third container, and adjusts and keeps the internal temperature of the third container to a fourth temperature lower than the third temperature.

In the EA3 step, amounts B3 and B3' of the compound in the third buffer solution are measured at a time interval t2.

On the basis of the amounts B3 and B3' and the time interval t2, an excretion rate of the compound by passive diffusion from the basal/basolateral face of the cells is determined.

The method determines a time-dependent change (slope of the graph) [4] in the EA step in Fig. 1. The test section for which B3 and B3' are measured (the compound from the third buffer solution is sampled) is Test Section 3 in Fig. 3.

Initially, the CE completion step operates by a procedure as above. Next, the EA3 step, which operates after the CE completion step, feeds a third buffer solution to the third container, and adjusts and keeps the internal temperature of the third container to a fourth temperature lower than the third temperature.

The third buffer solution is as in the Step EA2. The fourth temperature is such a temperature as to restrain cells which have taken up the compound from excreting the compound to the outside, and can be appropriately selected according to the cell type. In general, the fourth temperature is typically about 4°C (4°C ±1°C), and preferably 4°C.

In the EA3 step, amounts B3 and B3' of the compound in the third buffer solution are measured at a time interval t2. Specifically, the third buffer solution is sampled at least two times at the time interval t2 to measure the amounts of the compound in the third buffer solution. Although optimal time points of sampling vary depending on the target compound type and the cell type, the EA3 step may employ at least two, and preferably two or more time points at a time interval of typically several seconds to several tens of minutes. Multiple operations of sampling and compound amount measurement are as described above. In some cases, however, first sampling may be performed at the start of the step for the sake of convenience.

In Test Section 3, of the fraction of second basolateral efflux, the excretion of the compound via transporter (1) is restrained, and only the fraction by passive diffusion (2) is excreted (Fig. 3). Accordingly, analysis of the time-dependent change (slope [4] in Fig. 1) in the EA3 step by the procedure enables determination of the excretion rate of the compound by passive diffusion from the basal/basolateral face of the cells. Specifically, the excretion rate of the compound by passive diffusion from the basal/basolateral face of the cells is determined on the basis of the compound amounts B3 and B3' measured at the time interval t2 according to the formula: (B3 '-B3)/t2.

In still another embodiment, the method for kinetic analysis of a compound according to the present disclosure includes determining an excretion rate of the compound excreted from the apical face of the cell on the basis of the amounts B1 and B 1', the amounts B2 and B2', and the time interval t2.

Analysis of the difference in time-dependent change between the EA1 step and the EA2 step enables determination of the excretion rate of the compound excreted from the apical face of the cells. Specifically, the excretion rate of the compound from the apical face of the cells is determined on the basis of the compound amounts B1 and B1' and the compound amounts B2 and B2' each measured at the time interval t2, according to the formula: {(B1'-B2')-(B1-B2)}/t2.

In another embodiment, the method for kinetic analysis of a compound according to the present disclosure includes determining an excretion rate of the compound excreted via transporter from the basal/basolateral face of the cells, on the basis of the amounts B2 and B2', the amounts B3 and B3', and the time interval t2.

Analysis of the difference in time-dependent change between the EA2 step and the EA3 step enables determination of the excretion rate of the compound excreted via transporter from the basal/basolateral face of the cells. Specifically, the excretion rate of the compound excreted via transporter from the basal/basolateral face of the cells is determined from the compound amounts B2 and B2' and the compound amounts B3 and B3' each measured at the time interval t2, according to the formula: {(B2'-B3')-(B2-B3)}/t2.

In the description, the time interval between sampling operations in the EA step(s) is indicated as "t2". However, the EA1, EA2, and EA3 steps may employ the same time interval t2, or different time intervals t2. For example, assume that sampling is performed at different time points in the EA1, EA2, and EA3 steps. More specifically, assume that sampling is performed in the EA steps at time points $t2_i$, $t2_{ii}$, $t2_{iii}$, ... $t2_x$. To determine a time-dependent change (velocity or rate) in such a range as to be more approximal to a straight line after sampling and plotting of a graph on the basis of the measured amounts of the compound, the time-dependent change can be determined in the range from $t2_i$ to $t2_{ii}$ in the EA1 step, in the range from $t2_i$ to $t2_{iii}$ in the EA2 step, and in the range from $t2_{iv}$ to $t2_x$ in the EA3 step.

In another embodiment, the method for kinetic analysis of a compound according to the present disclosure further includes an uptake step before the CE step.

The uptake step exposes the cell to a solution containing the compound in a container or containers to allow the cell to take up the compound.

In the uptake step, amounts A and A' of the compound in the solution are measured at a time interval t3.

On the basis of the amounts A and A' and the time interval t3, an uptake rate at which the cell takes the compound therein is determined.

The method determines an initial uptake rate in the compound uptake step in Fig. 1. For S1, S2, and S3 indicated in Fig. 3, S1 ≈ S2 ≈ S3. Thus, the test section for which A and A' are measured (the compound in the solution is sampled) may be any of Test Sections 1 to 3 in Fig. 3.

As used herein, the term "compound uptake step" refers to the step of exposing a cell to a solution containing the compound to allow the cell to take up the compound (corresponding to Step 2 in the embodiments).

The initial uptake rate in the compound uptake step can be determined by any of several techniques. For example, the amount of the compound taken up by the cells may be determined after the CE step and the EA step, and after a subsequent IA step (intracellular assay for determining the amount of fraction remaining in cell) (after Steps 3 to 6 in the embodiments). Alternatively, Step 6 in the embodiments operates subsequent to Step 3, and, Step 6 operates on at least two wells at different time points, and the amounts of the compound taken up by the cells are determined. In another technique, a supernatant is sampled, and the amount of the compound taken up by the cells is determined, because the amount of the compound in the supernatant* decreases with time (medium loss).

In one embodiment, amounts A and A' of the compound in the solution are measured in the uptake step at a time interval t3. The compound amounts can be measured by a procedure as above.

Analysis of the time-dependent change in the uptake step enables determination of the uptake rate at which the cells take up the compound. Specifically, the uptake rate of the compound by the cells is determined on the basis of the compound amounts A and A' measured at the time interval t3 according to the formula: (A' -A)/t3.

The compound uptake step (Step 2 in the embodiments) preferably determines an initial concentration optimal for the compound. For example, initial concentrations are set, and graphs as in Fig. 1 are plotted on the basis of uptake data of the compound. The plotted graphs are compared, on the basis of which there is set an optimal initial concentration, namely, such a concentration as not to cause grave damage to the cell and as to enable exact data analysis (for example, such a concentration that the transporter is not saturated).

As described above, determination of the uptake rate of the compound by the cells, and the excretion rate of the compound from the cells enables easy and precise in-vitro analysis of kinetics of the compound, which analysis data can be easily understood and grasped. Determination of the velocities (rates) of kinetics of the compound can give the uptake amount and the excretion amount of the compound as visualized (graphed), and this more eases understanding of the compound kinetics and increases information for judgment or evaluation. For example, the configuration enables understanding of interplay between uptake and excretion of the compound, and/or anticipation of excretion after the measurement.

The present invention will be illustrated in further detail below with reference to several examples as specific embodiments thereof. It should be noted, however, that these examples (embodiments) are never construed to limit the scope of the present invention.

Embodiment 1

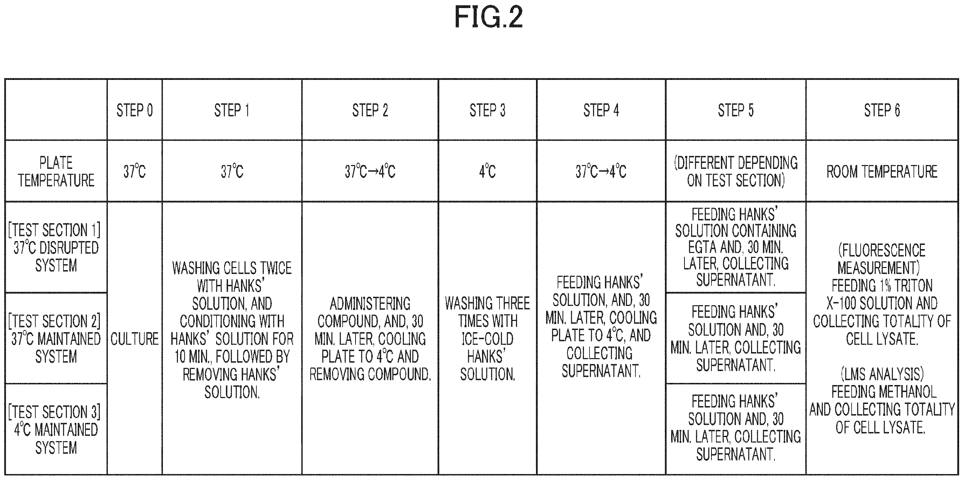

In this embodiment, a method for kinetic analysis of a compound to which the present invention is applied will be illustrated with reference to Steps 0 to 6 in Fig. 2. Steps 4 to 6 will be illustrated in detail with reference to Fig. 3.

The following embodiments are examples for illustrating the present invention, specific conditions such as buffer solution, temperature, and time indicated in these embodiments are for the illustration purpose, and other conditions having similar effects without departing from the technical idea of the present invention can be employed.

This embodiment describes a method using three test sections including Test Sections 1 to 3. Each test section contains a holding region for holding cells. These holding regions may include separate containers for the test sections, or may include one container which has been divided into holding regions for the test sections.

Step 0: Preparation and Culture of Cells

In this step, preparation and culture (incubation) of cells to analyze kinetics of a compound are initially performed. An example is illustrated below.

(1) Hepatocytes

Hepatocytes were prepared by in-situ collagenase perfusion method. The details are as follows. A rat (5-6 week-old) is anesthetized with pentobarbital and undergoes laparotomy, followed by insertion of a catheter into the portal vein and injection of a preperfusion solution (a $Ca^{2+}$ and $Mg^{2+}$ free Hanks' solution containing glycol ether diaminetetraacetic acid (EGTA)). After checking that blood is sufficiently removed from the liver, the perfusion is stopped. Perfusion is performed with the perfusate being changed to a collagenase solution. In this embodiment, the perfusion operates with a Hanks' solution containing 0.05% collagenase, but the collagenase solution is not limited to this. After checking that the tissue between the cells has been digested by collagenase, the perfusion is stopped. The liver is excised, sliced in a cooled Hanks' solution, and separated into cells by pipetting. Damaged hepatocytes are removed by centrifugation at 500 G for 5 minutes with an isotonic Percoll solution. The viability of the resulting hepatocytes is determined by the trypan blue-exclusion test. Hepatocytes having a viability of 85% or more are used for culture. Here, hepatocytes having a viability of 85% or more are used for culture, but such culture is not necessarily limited to the condition. In addition, the preparation of hepatocytes is not necessarily limited to that by in situ collagenase perfusion method. The hepatocytes to be used are not limited to those derived from a rat, and the strain of rat is not limited. This embodiment employs hepatocytes, but cells are not limited to this.

(2) Other Cells

Other than hepatocytes prepared from the liver typically of human or animal, other cells can also be applied to the experiment. Non-limiting examples of the other cells applicable to the experiment include humanized hepatocytes expressed typically in mice; iPS cell-derived hepatocytes resulting from induced differentiation from iPS cells; and cell lines (established cells) such as HepaRG cells and HepG2 cells. In addition, cells derived from various other organs are also applicable to the present invention.

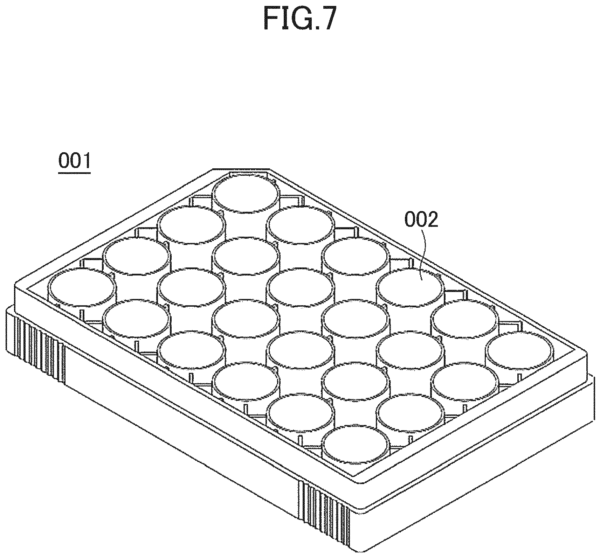

(3) Cells prepared by a procedure as above are suspended in a medium. The hepatocytes are suspended at a density of $5 \times 10^5$ cells/mL in the medium and plated onto a commercially available culture dish coated with collagen. The density upon plating, the medium, and the culture plate 001 are not limited. The culture plate is illustrated in Fig. 7. A 24-well culture plate having 24 culture regions (wells, 002) is here illustrated, but the culture plate is not limited to this, and any of containers in other shapes may be used as long as it can contain predetermined cells. After plating, culture is started under conditions of 5% $CO_2$ and 37°C using a $CO_2$ incubator. After 18 hours or more, the first medium change is performed. This embodiment employs a FCS- medium, which is a medium resulting from removal of FCS from the medium (10% FCS+) and supplementation with Matrigel, as the medium to be used for culture 18 hours after the plating. However, the medium can be any medium. After this, medium change with the medium (FCS-) is performed every 24 hours. As described later, testing is performed under three different conditions in Step 5 (Test Sections 1, 2, and 3; described in detail in Step 5), three independent culture plates under the same conditions are prepared at this point. In Steps 0 to 4 and Step 5, the same testing operations are performed in all the three test sections.

Step 1: Conditioning of Cells

In this step, the cells cultured in Step 0 are conditioned to a condition suitable for kinetic analysis. An example is illustrated below.

The culture supernatant of the cells cultured in Step 0 for 4 days is removed, and 400 μL of Hanks' solution is added as a buffer. The cells are incubated at 37°C for 10 minutes (Fig. 2, Step 1). The type and amount of buffer are not limited.

Preferably, the operations of Step 1 are repeated twice in total. By replacing the medium used in the culture of Step 0 with buffer solution (such as Hanks' solution) and adapting the cells to the buffer ingredients, they are conditioned for the accurate measurement and analysis in the downstream steps. The number of times Step 1 is repeated can be changed depending on the types of buffer solution and cells used.

Step 2: Administration of Compound

In this step, the target compound to be kinetically analyzed is administered to cells. An example is illustrated below.

The buffer is removed, and then 200 μL of 10 M carboxydichlorofluorescein (CDF; a fluorescent reagent) is fed to the well. The plate is incubated at 37°C for 30 minutes and then maintained at 4°C for 5 minutes (Fig. 2, Step 2). The type, concentration, and amount of the reagent are not limited. CDF emits fluorescence and can therefore be easily quantified as a model reagent with a plate reader. The amount of administration, 200 μL, was chosen so that the cells as a whole in the well would be immersed in the reagent. The concentration of CDF may be a concentration conventionally used for a fluorescence assay of cells. This concentration is preferably applied also as an amount of the reagent for the detection with a plate reader. Thirty (30) minutes of the incubation time was determined on the basis of the result of a preliminary examination that the time required for getting the equilibrium between uptake and excretion of a compound is 30 minutes. The plate temperature was lowered to 4°C after incubation for 30 minutes for the purpose of preventing the leakage of the administered compound from the cells. This is because lowering the temperature in the container restrains cells from excreting the compound.

As a result of thus lowering the temperature of cells to a predetermined temperature or lower, the compound remains in the cells and the leakage of the compound from the cells can be restrained. In this embodiment, the temperature is lowered to 4°C, but the temperature may be any temperature as long as the leakage of the administered compound from the cells can be restrained at the temperature. The technique for the restrainment is not limited to lowering the temperature, but can be any technique by which leakage of the administered compound from the cells can be restrained, for example, by administering an inhibitor. In any case, the temperature in Step 2 is lower than the plate temperature in Step 1. This is because the temperature for the conditioning in Step 1 is preferably a temperature that activates absorption and excretion of the compound by cells (e.g., 37°C), in contrast to Step 2.

The timing of the compound administration is not limited to that in this embodiment. For example, the compound may be administered on the day before or a few days before the testing day. In this case, the compound may be administered in Step 0, and Steps 1 and 2 can be omitted.

In Step 2, the medium is sampled from the well at different incubation time periods, namely, at two or more time points. For example, the medium is sampled from the well at time points T and T' (T+t3). The compound in the sampled medium is measured by a technique as described later. This determines amounts A and A' of the compound measured at a time interval t3 in the compound uptake step before the CE step (Step 4). The uptake rate of the compound by the cells is determined according to the formula: (A' -A)/t3. However, in some cases, the start point in time of the step may be applied to the time point T for the sake of convenience.

Step 3: Cell Washing

In this step, a portion of the compound other than that transferred into the cells in Step 3 is washed away. An example is illustrated below.

The cells were washed three times with 400 μL of ice-cold Hanks' solution while keeping the plate to 4°C (Fig. 2, Step 3). The purpose of keeping the plate to 4°C is the same as that described above. The amount of the Hanks' solution for washing was determined to be 400 μL for the purpose of removing medium ingredients remaining on the inner wall of the well because the amount of the medium in culture is 400 μL. The number of washing in general biochemistry assays is usually three times, which is adopted here. Conditions for the washing are not limited. This step removes anything other than the target compound to be measured, thus contributes to improved accuracy of the kinetic analysis in the downstream steps.

Step 4 (CE step): Collection of Fraction of Basolateral Efflux

As an index for compound kinetic analysis, it is effective to analyze whether the administered compound tends to remain in the cells for a long time or only for a short time. Therefore, this step aims to obtain the data for the index, and the compound is leaked from the cells in a predetermined time before evaluating the cells in the individual test sections in Step 5 described later. An example is illustrated below.

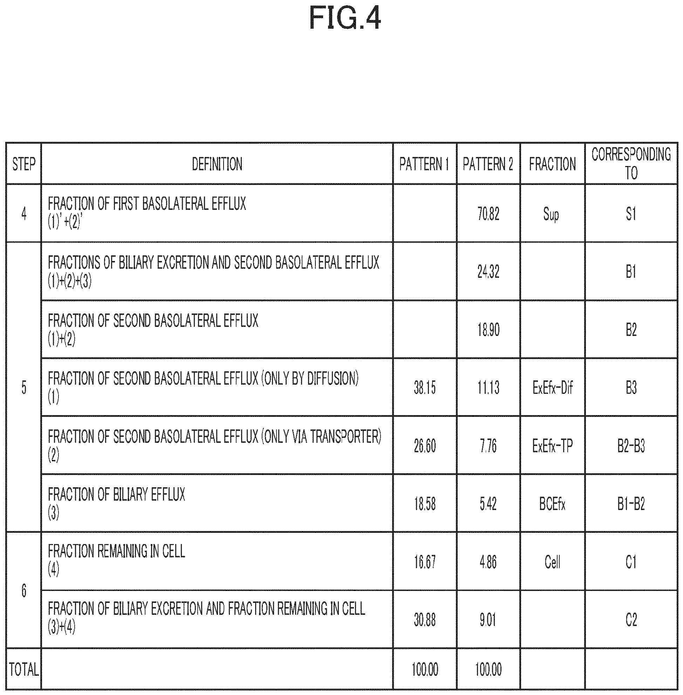

A buffer solution (such as Hanks' solution) for the pretreatment was first charged, the plate was incubated at 37°C for 30 minutes, and the Hanks' solution (supernatant) containing the compound was collected (Fig. 4, Step 4). The buffer solution may be the same as or different from the buffer solution used in Step 5. The plate was maintained at 37°C so that the compound accumulated in the cells (Fig. 3, 101 in the image view) by Step 3 is excreted into the Hanks' solution via the first basolateral efflux, that is, via the passively diffusion (Step 4 in Fig. 2, and (1)' in Fig. 3) and/or via the transporter (TP) (Step 4 in Fig. 2, (2)' and 102 in the image view in Fig. 3). Because basolateral efflux occurs also in Step 5 as described later, for the distinguishment, the basolateral efflux in Step 4 is defined as the first basolateral efflux, whereas the basolateral efflux in Step 5 is defined as the second basolateral efflux. Essentially, both steps represent the basolateral efflux (supernatant) from the cells. Transporters are membrane proteins that are responsible for transportation of substance and expressed on the cell membrane. They are responsible for active transportation of substance between inside and outside of the cell. The passive diffusion is excretion to outside of the cell other than that via the transporter and includes the leakage through the cell membrane. The compound excreted into the Hanks' solution in Step 4 was defined as a fraction of basolateral efflux, because the upper surface of the cells facing the Hanks' solution corresponds to the basal/basolateral face (Fig. 3, 104 in the image view), which is considered to be a part facing the blood vessel in the body.

Step 5, which will be described next, includes collecting the compound in three different operations (Test Sections 1, 2, and 3) and an index for examining whether the compound tends to remain in the cell can be acquired by, prior to Step 5, obtaining the fraction of first basolateral efflux (the compound excreted into Hanks' solution) in Step 4 as mentioned above.

The Hanks' solution used to obtain the fractions of the basolateral efflux may be of any test section. For example, Hanks' solutions of all Test Sections 1 to 3 described later, or a part thereof, may be evaluated.

In Step 4, the medium is sampled from the well at different incubation time periods, namely, at multiple time points. For example, the medium is sampled from the well at time points T and T' (T+t1). The compound in the sampled medium is measured by a technique as described later. This gives amounts S and S' of the compound as measured at a time interval t1 in the CE step (Step 4), from which an excretion rate of the compound from the basal/basolateral face of the cells is determined according to the formula: (S'-S)/t1. However, in some cases, the start point of the step may be applied to the time point T, for the sake of convenience.

The Steps 0 to 4 are performed in at least one of Test Sections 1 to 3, as shown in Fig. 2. In Step 5, operations are performed under different conditions for Test Sections 1 to 3 to obtain multiple index data sought in the present invention by the analysis described later. An example is illustrated below.

Step 5, Test Section 1 (EA1 step): Collection of Supernatant in 37°C Disrupted System

In Test Section 1, as illustrated in Test Section 1 in Fig. 3, the compound is excreted from the cell into a buffer solution (such as Hanks'(-) solution) in an amount equal to the sum of biliary efflux (3) and the second basolateral efflux (that is, diffusion (1) and excretion via transporter (TP) (2)) discharge. Hanks'(-) solution is Hanks' solution free of calcium and magnesium ions and used, for example, when cell-cell adhesion is not enhanced as in this test section. For the purpose of positively removing cell-cell adhesion, a chelator such as EGTA is used as described later. Next, 200 μL of Hanks'(-) solution containing 1 mM EGTA was added. EGTA has a chelating effect of restraining the effect of $Ca^{2+}$ and $Mg^{2+}$ involved in the adhesion of intercellular adhesion molecules and is a reagent for removing cell-cell adhesion. This causes the disruption of bile ducts formed during the culture process. The test section was incubated at 37°C for 30 minutes, from which the supernatant was collected. The chelating reagent is not limited to EGTA, as long as it has a chelating effect. Type and amount of the buffer containing EGTA and the temperature and time of the incubation are not limited. In Test Section 1 of Step 5, the compound remaining in the cells (Fig. 3, Step 5, and (1) and (2) in the image view) and the compound excreted into the bile canaliculi (Fig. 3, Step 5, and (3) in the image view) upon the completion of Step 4 are all excreted into the supernatant and collected (Fig. 3, Step 5, Test Section 1). In Step 5, (3) is defined as a biliary-excreted fraction, because the cell membrane portion around the gap formed in the cell-cell adhesion portion corresponds to an apical face (Fig. 3, 105) and the gap is assumed to be a bile canaliculus (Fig. 3, 103). In Test Section 1 in Step 5, the temperature is maintained at 37°C, and therefore the fractions excreted from the cells basolaterally include the fraction via passive diffusion (Fig. 3, Step 5, (1)) and the fraction via the transporter (Fig. 3, Step 5, (2)).

In Test Section 1 (EA1 step) in Step 5, the medium is sampled from the well at different incubation time periods, namely, at multiple time points. For example, the medium is sampled from the well at time points T and T' (T+t2). The compound in the sampled medium is measured by a procedure as described later. This determines the amounts B1 and B1' of the compound as measured at the time interval t2 in the EA1 step. On the basis of the amounts, the excretion rate of the compound excreted from the basal/basolateral face and the apical face of the cell is determined according to the formula: $(B1'-B1)/t2$. However, in some cases, the start point of the step may be applied to the time point T, for the sake of convenience.

Step 5, Test Section 2 (EA2 step): Collection of Supernatant in 37°C Maintained System

In Test Section 2, bile ducts are not disrupted, unlike Test Section 1, and the compound is excreted only by the second basolateral efflux (diffusion (1) and transporter (2)) indicated in Fig. 3.

Hanks' solution was fed in an amount of 200 μL. Test Section 2 is devoid of any chelating agent such as EGTA, unlike Test Section 1, and therefore cell-cell adhesion is maintained. Accordingly, Test Section 2 is a test section where the disruption of bile canaliculi is not induced. The test section was incubated at 37°C for 30 minutes, from which the supernatant was collected. In Test Section 2 in Step 5, the compound remaining in the cells (Step 5, and (1) and (2) in the image view in Fig. 3) upon the completion of Step 4 is excreted into the supernatant and collected (Step 5, Test Section 2 in Fig. 3). Following the description, the compound excreted into the supernatant is defined as a fraction of second basolateral efflux. In Test Section 2 in Step 5, the temperature is maintained at 37°C, and therefore the fractions excreted from the cells basolaterally include the fraction via passive diffusion (Fig. 3, Step 5, (1)) and the fraction via the transporter (Fig. 3, Step 5, (2)).

In Test Section 2 (EA2 step) in Step 5, the medium is sampled from the well at different incubation time periods, namely, at multiple time points. For example, the medium is sampled from the well at time points T and T' (T+t2). The compound in the sampled medium is measured by a procedure as described later. On the basis of this, the amounts B2 and B2' of the compound as measured at the time interval t2 is determined in the EA2 step, from which the excretion rate of the compound excreted from the basal/basolateral face of the cells is determined according to the formula: $(B2'-B2)/t2$. However, in some cases, the start point of the step may be applied to the time point T, for the sake of convenience.

Each of the amount of the compound in the fraction of biliary efflux (3) and the amount of the compound in the fraction of second basolateral efflux can be determined by quantitatively analyzing the amounts of the compound collected from Test Section 1 and Test Section 2 in Step 5, for example, by subtracting the quantified value of the amount of the compound collected in Test Section 2 from the quantified value of the amount of the compound collected in Test Section 1.

Step 5, Test Section 3 (EA3 step): Collection of Supernatant in 4°C Maintained System

Test Section 3 has a temperature condition lower than that in Test Sections 1 and 2 as described later, and thereby the excretion of the compound via the transporter (1) is restrained, and only a portion of the compound excreted by diffusion (2) is excreted in the fraction of second basolateral efflux.

Hanks' solution was fed in an amount of 200 μL. The test section was incubated at 4°C for 30 minutes, from which the supernatant was collected. The testing temperature is different from that in Test Section 2 in Step 5, which is also a maintained system. This aims to restrain basolateral efflux via the transporter by cooling to 4°C. Test Section 3 in Step 5 employs a test section having a low temperature of 4°C to restrain the transporter activity, and to thereby quantify only the amount excreted by passive diffusion (Fig. 3, Step 5, (1)). Comparing this with Test Section 2 in Step 5 enables determination (quantification) of the amount of the basolateral efflux fraction via transporter. For each compound, a specific transporter is present for moving between inside and outside of the cell. Therefore, the ability to exclude the amount of excretion by passive diffusion and to quantify the amount of excretion via the transporter means the ability to quantify the amount of excretion specific for the compound. Type and amount of the buffer and the temperature and time of the incubation are not limited. As described above, the fraction excreted via the transporter can be determined by quantifying the compound collected from Test Section 2 and Test Section 3 in Step 5.

In Test Section 3 (EA3 step) of Step 5, the medium is sampled from the well at different incubation time periods, namely, at multiple time points. For example, the medium is sampled from the well at time points T and T' (T+t2). The compound in the sampled medium is measured by a procedure as described later. On the basis of this, the amounts B3 and B3' of the compound as measured at a time interval t2 are determined, from which the excretion rate of the compound excreted by passive diffusion from the basal/basolateral face of the cells is determined according to the formula: $(B3'-B3)/t2$. However, in some cases, the start point of the step may be applied to the time point T, for the sake of convenience.

Although the determination of excreted fractions using Test Sections 1, 2, and 3 is illustrated in this embodiment, the number of test sections is not limited to three. For example, when only the separation between biliary efflux (3) and the second basolateral efflux ((1)+(2)) is desired, it is sufficient to conduct only Test Sections 1 and 2; and when only the separation between the diffusion (1) and the transporter (1) in the second basolateral efflux is desired, it is sufficient to conduct only Test Sections 2 and 3. It is also possible to measure only the amount excreted by diffusion by additionally having a disrupted system at 4°C, as needed, to restrain biliary efflux in excretion via transporter. For example, comparison of this 4°C disrupted system with Test Section 3 enables separate evaluation of the biliary efflux by diffusion and the biliary efflux via transporter.

In addition, by determining the difference between Test Section 1 (EA1 step) and Test Section 2 (EA2 step), the amount of the compound biliary-route excreted per unit time can be determined. Specifically, the amount is determined with the denominator being the time period (herein, the time interval t2) between the time points T and T' (T+t2), and with the numerator being the difference in the amount of the compound between the time points (namely, the amount of biliary efflux). For example, the biliary excreted amount, namely, the excretion rate of the compound from the apical face of the cells can be determined according to the formula: $\{(B1'-B2')-(B1-B2)\}/t2$. However, in some cases, the start point of the step may be applied to the time point T, for the sake of convenience.

By determining the difference between Test Section 2 (EA2 step) and Test Section 3 (EA3 step), the amount of the compound excreted per unit time into blood via transporter can be determined. Specifically, the amount is determined with the denominator being the time period (herein, the time interval t2) between the time points T and T' (T+t2) and with the numerator being the difference in amount of the compound between the time points (namely, the amount of the compound excreted into the blood via transporter). For example, the amount of the compound excreted into the blood via transporter, namely, the excretion rate of the compound from the basal/basolateral face of the cells via transporter can be determined according to the formula: $\{(B2'-B3')-(B2-B3)\}/t2$. However, in some cases, the start point of the step may be applied to the time point T, for the sake of convenience.

The steps in each of Test Sections 1 to 3 may be performed in any sequence, or may be performed in parallel.

Step 6: Collection of Biliary Fraction and Intracellular Fraction

In Step 6, the same operations are performed for the three test sections again. Step 6 conducts the compound collection for quantifying the fraction remaining in the cells. For example, when fluorescent agent is quantified using a plate reader, it is preferred that, for example, 200 μL of a Hanks solution containing 1% surfactant is added and suspended, and the totality is collected (Fig. 2, Step 6). This disrupts the cell membrane and allows the compound remaining in the cells to be excreted. The resulting samples were transferred to a culture plate, followed by fluorescence measurement using a plate reader. Wells containing only the Hanks' solution are prepared for the blank measurement. Fluorescence intensity is measured at an excitation wavelength of 484 nm and an absorption wavelength of 519 nm.

When the compound is quantified using a mass spectrometer (liquid chromatograph-mass spectrometer; LCMS), water is added for lowering the tonicity, the fraction remaining in the cells is extracted by a treatment with an organic solvent and then suspended in an organic solvent such as methanol, and the totality is collected (Fig. 2, Step 6). Quantification of the compound is subsequently performed using an LCMS as described later.

Determination of Distribution Ratio and Scoring of Respective Fractions Based on Measurement Results

The distribution ratio to the fractions is determined on the basis of the fluorescence intensities reflecting the amounts of the compound obtained in the steps. Here, the sum of Steps 5 and 6 (Fig. 3, B#+C#=(1)+(2)+(3)+(4)) is defined as 100% amount of the compound. This is referred to as "pattern 1."

From the six values (B1, B2, B3, C1, C2, and C3) obtained by the measurement of fluorescent amount, the distribution ratio of fractions illustrated in pattern 1 in Fig. 4 is obtained. As for these values,

the fraction of the second basolateral efflux only by diffusion (extracellular efflux by diffusion; ExEfx-Dif) is expressed as B3;
the fraction of the second basolateral efflux only by the transporter (extracellular efflux by transporter; ExEfx-TP) is expressed as B2-B3;
the fraction of the biliary efflux (bile canaliculi efflux; BCEfx) is expressed as B1-B2; and
the fraction remaining in the cells (Cell) is expressed as C1.

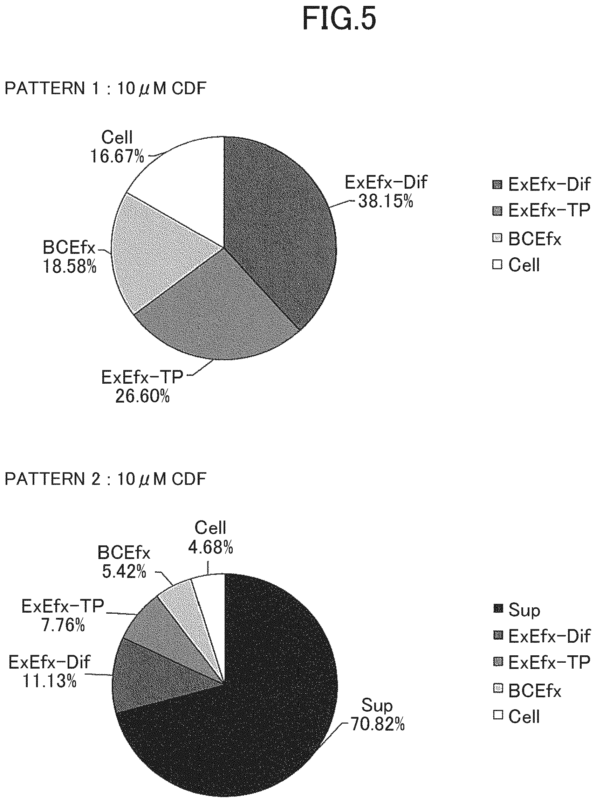

Based on this result, a circle graph such as pattern 1 in Fig. 5 can be drawn, and this enables visual understanding of an overview of the distribution ratio of the fractions.

Furthermore, the quantification result enables scoring specific to the compound as follows. In this embodiment, the scoring determined for CDF is illustrated. The determined scores are not limited to these.

A score for evaluating basolateral efflux via transporter is determined:
according to (B2-B3)/B2, as the ratio of the amount of the compound excreted via transporter to the amount of the compound of the fraction of second basolateral efflux (ratio of extracellular efflux by diffusion; RexEMTP).

A score for evaluating the biliary efflux can be determined:

according to (B1-B2)/(C1+C2), as the ratio of the amount of the compound excreted into the bile canaliculi to the totality of the compound taken up into the cells (biliary retention drug, BIRD),
or alternatively, for example,
according to (C2-C1)/C2 or (B1-B2)/C1, as the ratio of the amount of the compound excreted into bile canaliculi to the amount of the compound remaining in the cells.

Comparison of Distribution Ratios and Scores Between Different Compounds

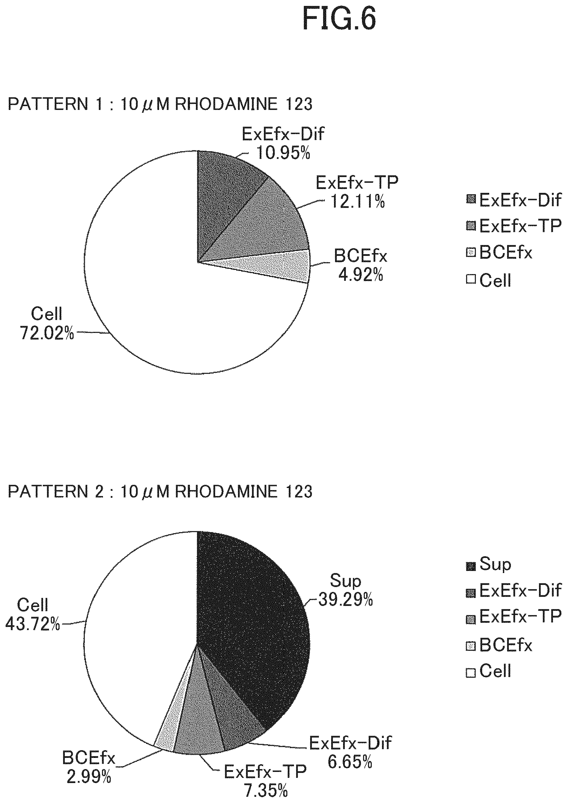

The operations same as those described above can be performed using Rhodamine 123 instead of CDF to obtain the result shown in Fig. 6 (pattern 1). The result indicates that a distribution ratio apparently different from the result of CDF shown in Fig. 5 (pattern 1) can be detected.

For example, the ratio of the amount of compound excreted via transporter to the amount of compound excreted basolaterally (RexEMTP) and the ratio of the amount of compound excreted into the bile duct to the totality of compound taken up into the cells (BiRD) for CDF are respectively, 41.08 and 18.58, while those for Rhodamine 123 are respectively 52.52 and 4.92, indicating that CDF is a compound that has a stronger tendency to be excreted into the bile canaliculi but not basolaterally than Rhodamine 123 does. As described above, the present invention enables evaluation of compounds for the pharmacokinetics they exhibit.

Visualization of Compound Kinetics

On the basis of the uptake amount and excretion amount of the compound determined by the procedure as above, the kinetics of the compound in cells may be graphed as a compound uptake-excretion curve (disposition curve) as illustrated in Fig. 1. This enables visual understanding of kinetics of the compound.

Embodiment 2

Embodiment 2 describes the determination of distribution ratio and scoring of fractions on the basis of the measurement results, where the determination is performed by a procedure different from Embodiment 1.

In this embodiment, a quantified value S# (corresponding to Step 4) described in Fig. 3, in addition to the distribution ratio of the fractions shown in pattern 1 in Embodiment 1, is used to obtain information on whether the administered compound has a strong or weak tendency to remain in the cells, which enables more accurate evaluation. Accordingly, the sum of steps 4, 5, and 6 (Fig. 3, S#+B#+C#=(1)'+(2)'+(1)+(2)+(3)+(4)) is defined as the 100% amount of the administered compound. This is referred to as "pattem 2". Accordingly, nine values (S1, S2, S3, B1, B2, B3, C1, C2, and C3) obtained in the test sections in steps 4, 5, and 6 can be used as evaluation indices.

The nine values obtained from measurement of fluorescent amounts give the distribution ratio of the fractions shown in pattern 2 in Fig. 4. As for these values:

the fraction of the first basolateral efflux (Sup fraction) is expressed as S1 (≈ S2 ≈ S3);
the fraction of the second basolateral efflux excreted only by diffusion (extracellular efflux by diffusion, ExEfx-Dif) is expressed as B3;
the fraction of the second basolateral efflux excreted only by the transporter (extracellular efflux by transporter, ExEfx-TP) is expressed as B2-B3;
the fraction of the biliary efflux (bile canaliculi efflux, BCEfx) is expressed as B1-B2; and
the fraction remaining in the cells (Cell) is expressed as C1.

On the basis of the results, a circle graph such as pattern 2 in Fig. 5 can be drawn, and this enables visual understanding of an overview of the distribution ratio of the fractions. As determined by a procedure described in Embodiment 2, the Sup fraction, which is an index for tendency to remain or not to remain in the cells, for CDF is 70.82, while that for Rhodamine 123 is 39.29 (Fig. 6, pattern 2), indicating that CDF has a tendency to be excreted basolaterally. This is consistent with the evaluation of the fraction remaining in the cells in Embodiment 1.

Furthermore, the quantification results enable scoring specific to the compound, as described in Embodiment 1.

Embodiment 3

This embodiment makes analysis with a reference (100%) being the amount of the compound in cells at the time point of transferring to EA step (pattern 3). Although the amounts of compound in Test Sections 1 to 3 should be principally identical, there occurs experimental errors, and the analysis is made on the basis of the average. The amounts of compound in the individual fractions shown in pattern 1 in Embodiment 1 are substituted into the following formula:

$$\text{Compound amount } 100\% = \{(B1+C1)+(B2+C2)+(B3+C3)\}/3$$

The pattern 3 enables more precise analysis than that in pattern 1.

Embodiment 4

This embodiment makes analysis with a reference (100%) being the total uptake amount of the compound into cells (pattern 4). Although the amounts of compound in Test Sections 1 to 3 should be principally identical, there occurs experimental errors, and the analysis is made on the basis of the average. The amounts of compound in the fractions shown in pattern 2 in Embodiment 2 are substituted into the following formula:

$$\text{Compound amount } 100\% = \{(S1+B1+C1)+(S2+B2+C2)+(S3+B3+C3)\}/3$$

The pattern 4 enables more presence analysis than that in pattern 2.

Embodiment 5

Embodiment 5 describes an example of apparatus automating a series of steps described in Embodiments 1 to 4. Regarding purposes of operations and functions of configurations of the apparatus described later, descriptions may be omitted when the descriptions overlap those of the aforementioned Embodiments 1 to 4.

In the configurations of the apparatus described later, the regions holding cells referred to as Test Sections 1 to 3 illustrated in Embodiments 1 to 4 are described using expressions such as "a well for Test Section 1," "a well for Test Section 2," and "a well for Test Section 3." For example, containers in the "well culture plate" may be defined for respective test sections, or wells in a well culture plate may be divided and the divided regions may be defined as, for example, "a first container," "a second container," and "a third container."

As for the well for Test Section 1 and the well for Test Section 2 in particular, effective evaluation may be provided in terms of accuracy and speed by defining them as wells in divisions of one well culture plate because they undergo a substantially the same temperature control.

As for the operations of the apparatus described latter, exchange of wells (containers) for respective test sections may operate automatically or manually. Descriptions on exchange and placement of the containers will be omitted in the following description.

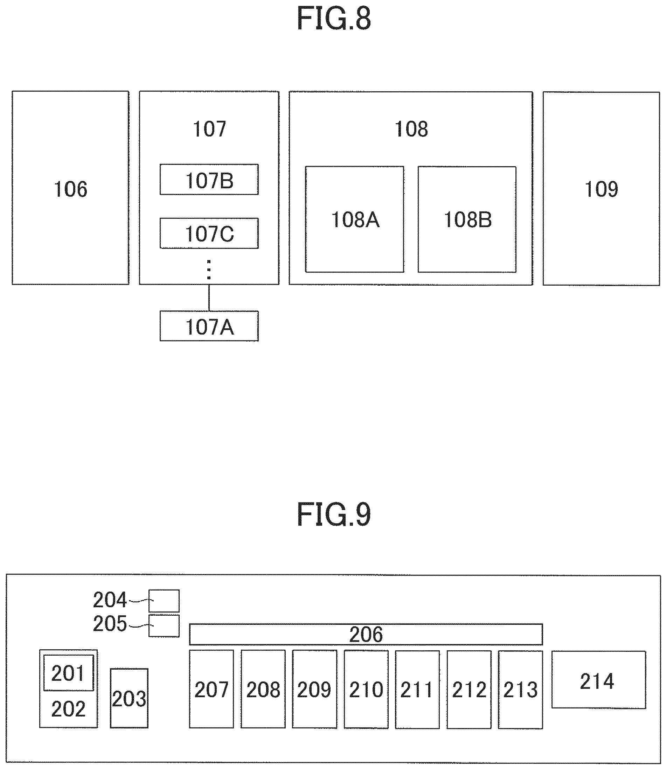

The apparatus includes a culture unit 106, a sample preparation unit 107 (including an input unit 107A), an analyzing unit 108, and a display unit 109, as illustrated in Fig. 8. The sample preparation unit 107 has, for example, a temperature control unit 107B for controlling temperature in individual containers (plates) described later, and a liquid-feeding unit 107C capable of supplying or collecting liquid to or from a container. The analyzing unit 108 has a measurement unit 108A for measuring the amount of a component such as a compound, and an analyzer 108B for analyzing each of: an amount excreted via transporter, an amount excreted via the bile duct, an amount remaining in the cells, and an amount excreted via a route (diffusion) other than the transporter and the bile duct on the basis of the amount of the component such as a compound obtained in the measurement unit; and analyzing an excretion rate and/or an uptake rate on the basis of measurement results at multiple time points. The above configuration of the apparatus is an example and, for example, any other configurations may be adopted, such as one in which only the operation of the analyzer is performed in another apparatus and the information obtained by the measurement unit is transmitted to the other apparatus.

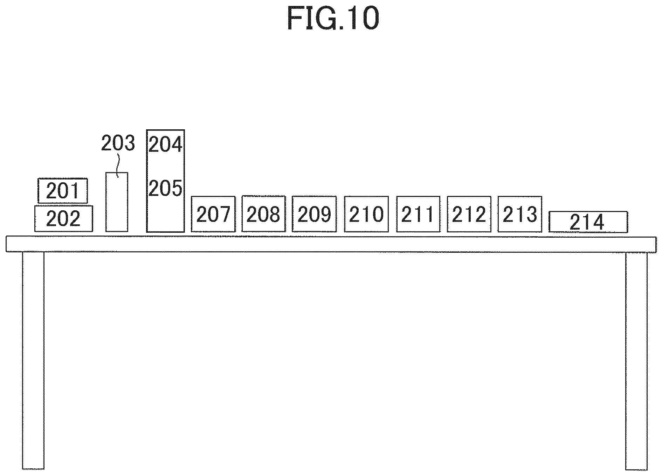

A detailed configuration of the sample preparation unit is shown in Figs. 9 and 10. The sample preparation unit aims to prepare fractions to be analyzed automatically as described in Embodiments 1 to 4. Components will be described in connection with the flow chart described later.

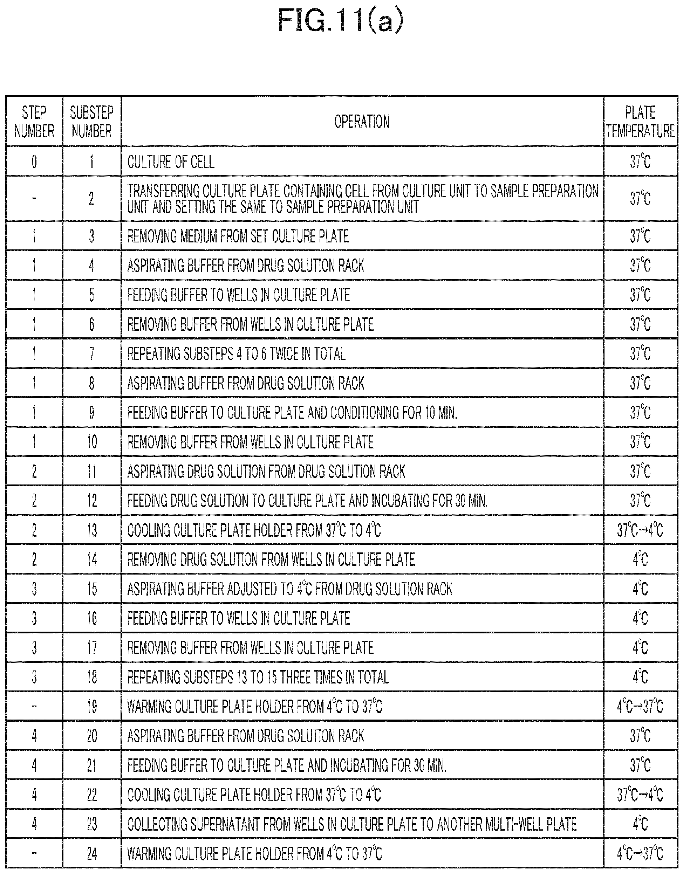

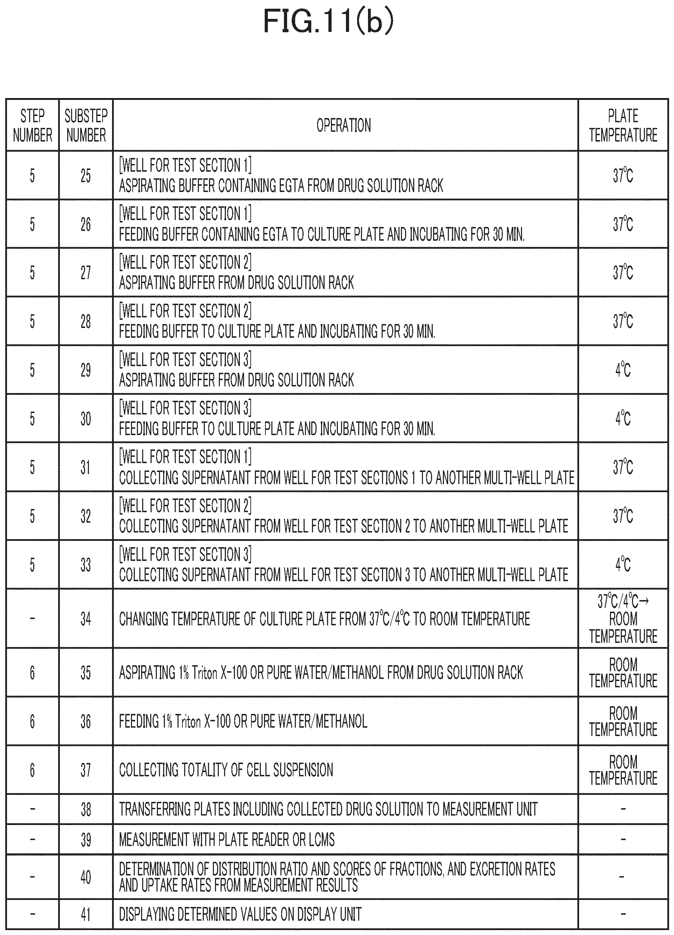

Fig. 11 is a flow chart of operations of the automatic measuring apparatus. The flow chart of Fig. 11 is merely an example, and the operations may be different from this when a timing of the compound administration is different as described in Embodiment 1, Step 2. In this embodiment, plates having a plurality of cell holding regions (wells) are used as an example for illustrating the containers for holding cells, but the containers are not limited to plates, and any containers that can contain cells may be used.

Transfer from Culture Unit to Sample Preparation Unit

Initially, hepatocytes are cultured in the culture unit 106 (Figs. 8 and 11) (Fig. 11(a), Substep 1). Subsequently, the plates containing the cultured hepatocytes are transferred on a first thermostat plate holder 210 and a second thermostat plate holder 211 in the sample preparation unit (Figs. 8 and 11) (Fig. 11(a), Substep 2).

Sample Preparation: Corresponding to Step 1 in Embodiments 1 to 4

An aspiration head 205, equipped with an aspiration nozzle to aspirate liquid and installed in the liquid-feeding unit 107C, moves to a well 002, filled with medium to be removed, in a culture plate 001 on the plate holder and aspirates the medium from the well to remove the totality of the medium (Fig. 11(a), Substep 3). The removed medium is collected (discarded) into the waste liquid tank 206.
Next, a tip for containing liquid is attached from a tip rack 207 storing tips onto a tip head 204 installed to an aspiration nozzle. The tip head moves to a room-temperature drug solution rack 209 and aspirates a buffer (Fig. 11(a), Substep 4). The tip head moves to a target well, introduces the buffer (Fig. 11(a), Substep 5), then moves to a dust box 214, and discards the tip. An exchangeable tip is used here to eliminate or minimize contaminations, but the tip is not limited to this. A syringe pump controller 201 controls a syringe pump 203 and thereby controls the tip head 204 and the aspiration head 205. A temperature controller 202 controls the temperatures of the first thermostat plate holder 210 and the second thermostat plate holder 211, and of the culture unit.
The aspiration head moves to the well filled with the buffer and removes the buffer (Fig. 11(a), Substep 6). The removed medium (buffer) is discarded in the waste liquid tank 206. This process is repeated twice in total (washing step) (Fig. 11(a), Substep 7).
Next, after being attached with a tip in the tip rack 207, the tip head 204 moves to the room-temperature drug solution rack 209, and aspirates the buffer (Fig. 9, Substep 8). The tip head moves to the target well, introduces the buffer, then moves to the dust box 214, and discards the tip. The plate stands at 37°C for 10 minutes (conditioning) (Fig. 11(a), Substep 9). Subsequently, the aspiration head 205 moves to the target well filled with the buffer and removes the totality of the buffer (Fig. 11(a), Substep 10).

Sample Preparation: Corresponding to Step 2 in Embodiments 1 to 4

After being attached with a tip in the tip rack 207, the tip head 204 moves to the room-temperature drug solution rack 209, and aspirates the drug solution (Fig. 11(a), Substep 11). The tip head 204 moves to the target well and introduces the drug solution (Fig. 11(a), Substep 12), and then moves to the dust box 214 and discards the tip. The plate stands at 37°C for 30 minutes (Fig. 11(a), Substep 12). Subsequently, the temperatures of the first thermostat plate holder 210 and the second thermostat plate holder 211, which are an example configuration of the container holding unit for holding containers, change from 37°C to 4°C (Fig. 11(a), Substep 13), and then the aspiration head 205 moves to the well filled with the drug solution and removes the totality of the drug solution (Fig. 11(a), Substep 14).
Although the temperature control unit in this embodiment is described as a plate folder having a temperature controlling function (thermostat plate holder), the temperature control unit may be provided separately from the container holding unit.

Sample Preparation: Corresponding to Step 3 in Embodiments 1 to 4

After being attached with a tip in the tip rack 207, the tip head 204 moves to a refrigerated drug solution rack 208, and aspirates a buffer (Fig. 11(a), Substep 15). A refrigerated drug solution is used to stop active biological phenomena such as the transporter activity, as described above. For this purpose, the refrigerated drug solution rack 208 aims to hold, for example, a drug solution or a buffer at a low temperature. The tip head moves to the target well, introduces the buffer (Fig. 11(a), Substep 16), then moves to the dust box 214, and discards the tip. The aspiration head moves to the well filled with the buffer and removes the buffer (Fig. 11(a), Substep 17). The removed medium is discarded in the waste liquid tank 206. This process is repeated three times in total (washing step) (Fig. 11(a), Substep 18).

Sample Preparation: Corresponding to Step 4 in Embodiments 1 to 4

The temperatures of the first thermostat plate holder 210 and the second thermostat plate holder 211 change from 4°C to 37°C (Fig. 11(a), Substep 19). After being attached with a tip in the tip rack 207, the tip head 204 moves to a refrigerated drug solution rack 208 and aspirates the buffer (Fig. 11(a), Substep 20). The tip head 204 moves to the target well, introduces the buffer (Fig. 11(a), Substep 21), then moves to the dust box 214, and discards the tip. The plate stands at 37°C for 30 minutes (Fig. 11(a), Substep 20). Subsequently, the temperatures of the first thermostat plate holder 210 and the second thermostat plate holder 211 change from 37°C to 4°C (Fig. 11(a), Substep 22). After being attached with a tip in the tip rack 207, the tip head 204 moves to the well filled with the buffer containing the compound, aspirates the buffer (supernatant) containing the compound, and dispenses it into collection plates for collection on a first plate holder 212 and a second plate holder 213 (collection) (Fig. 11(a), Substep 23).

Sample Preparation: Corresponding to Step 5 in Embodiments 1 to 4

The temperature of the first thermostat plate holder 210 changes from 4°C to 37°C (Fig. 11(a), Substep 24). After being attached with a tip in the tip rack 207, the tip head 204 moves to the room-temperature drug solution rack (209) and aspirates a buffer containing EGTA (Fig. 11(a), Substep 25). The tip head 204 moves to a well for Test Section 1 on the first plate holder 212, introduces the buffer containing EGTA (Fig. 11(b), Substep 26), then moves to the dust box 214, and discards the tip. The plate stands at 37°C for 30 minutes (Fig. 11(b), Substep 26).
After being attached with a tip in the tip rack 207, the tip head 204 moves to the room-temperature drug solution rack 209, and aspirates a buffer (Fig. 11(b), Substep 27). The tip head 204 moves to a well for Test Section 2 on the first plate holder 212, introduces the buffer (Fig. 11(b), Substep 28), then moves to the dust box 214, and discards the tip. The plate stands at 37°C for 30 minutes (Fig. 11(b), Substep 28).
After being attached with a tip in the tip rack 207, the tip head 204 moves to the refrigerated drug solution rack 209, and aspirates the buffer (Fig. 11(b), Substep 29).
The tip head 204 moves to a well for Test Section 3 on the second plate holder 213, introduces the buffer (Fig. 11(b), Substep 30), then moves to the dust box 214, and discards the tip. The plate stands at 4°C for 30 minutes (Fig. 11(b), Substep 30).
After being attached with a tip in the tip rack 207, the tip head 204 moves to the well filled with an EGTA buffer containing the compound, aspirates the EGTA buffer (supernatant) containing the compound, and dispenses (collects) the buffer into a collection plate for collection on the first plate holder 212 (Fig. 11(b), Substep 31).
After being attached with a tip in the tip rack 207, the tip head 204 moves to the well filled with the buffer containing the compound and aspirates the buffer (supernatant) containing the compound and dispenses (collects) the buffer into a collection plate for collection on the first plate holder 212 (Fig. 11(b), Substep 32).
After being attached with a tip in the tip rack 207, the tip head 204 moves to the well filled with the EGTA buffer containing the compound, aspirates the buffer (supernatant) containing the compound, and dispenses (collects) the buffer into the collection plate for collection on the first plate holder 213 (Fig. 11(b), Substep 33).
The temperatures of the first thermostat plate holder 210 and the second thermostat plate holder 211 both change to room temperature (Fig. 11(b), Substep 34). After being attached with a tip in the tip rack 207, the tip head 204 moves to the room-temperature drug solution rack 209 and aspirates 1% TritonX-100 or pure water/methanol (Fig. 11(b), Substep 35). The tip head 204 moves to wells for Test Sections 1, 2, and 3 on the first thermostat plate holder 210 and the second thermostat plate holder 211, and introduces 1% TritonX-100 or pure water/methanol (Fig. 11(b), Substep 36), then moves to the dust box 214, and discards the tip.
After being attached with a tip in the tip rack 207, the tip head 204 moves to a well filled with the reagent, aspirates the totality of the cell suspension, and dispenses the suspension into the collection plates for collection on the first plate holder 212 and the second plate holder 213 (collection) (Fig. 11(b), Substep 37).
The operations of the apparatus have been described provided that the operations of Test Sections 1 to 3 in Embodiments 1 to 4 are performed in sequence. However, the steps may be performed in sequence or in parallel.

Transfer from Sample Preparation Unit to Measurement Unit

The compound collected from the culture plate transfers to the measurement unit (Fig. 11(b), Substep 38). In the measurement unit, the measurement of the compound operates with a plate reader or LCMS (Fig. 11(b), Substep 39).
Determination of Distribution Ratio and Score and Display of Result by Analysis Unit
The distribution ratio and the scores of the fractions, and the excretion rates and the uptake rates are determined on the basis of the measurement results (Fig. 11(b), Substep 40). Then, a display unit displays the resulting determined values (Fig. 11(b), Substep 41).

List of Reference Signs

001    culture plate
002    well
101    hepatocyte
102    transporter
103    bile canaliculus
104    basal/basolateral face
105    apical face
201    syringe pump controller
202    temperature control unit
203    syringe pump
204    tip head
205    aspiration head
206    liquid waste tank
207    tip rack
208    refrigerated drug solution rack
209    room-temperature drug solution rack
210    firstthermostat plate holder
211    second thermostat plate holder
212    first plate holder
213    second plate holder
214    dust box

**Claims**

1. A method for kinetic analysis of a compound using a cell, the method comprising:

   a CE step of feeding a first buffer solution to a container containing a target compound and housing a cell, adjusting and keeping an internal temperature of the container to a first temperature, and adjusting the internal temperature of the container to a second temperature lower than the first temperature,
   the method further comprising:

   measuring amounts S and S' of the compound in the first buffer solution at a time interval t1 in the CE step; and
   determining an excretion rate of the compound excreted from a basal/basolateral face of the cell based on the amounts S and S' and the time interval t1.

2. The method according to claim 1 for kinetic analysis of a compound,
   wherein the method comprises:

   conducting the CE step separately on a first container, a second container, and a third container each housing the cell and containing the compound;
   a CE completion step of collecting the first buffer solution separately from the first container, the second container, and the third container; and
   an EA1 step, after the CE completion step, of feeding a second buffer solution containing a reagent for removing cell-cell adhesion to the first container, and adjusting and keeping an internal temperature of the first container to a third temperature, and
   wherein the method further comprises:

   measuring amounts B1 and B1' of the compound in the second buffer solution at a time interval t2 in the EA1 step; and
   determining an excretion rate of the compound excreted from the basal/basolateral face and an apical face of the cell based on the amounts B1 and B1' and the time interval t2.

3. The method according to claim 2 for kinetic analysis of a compound,
   wherein the method further comprises:

   an EA2 step, after the CE completion step, of feeding a third buffer solution to the second container, and adjusting and keeping the internal temperature of the second container to the third temperature, and

wherein the method further comprises:

measuring amounts B2 and B2' of the compound in the third buffer solution at the time interval t2 in the EA2 step; and
determining an excretion rate of the compound excreted from the basal/basolateral face of the cell based on the amounts B2 and B2' and the time interval t2.

4.  The method according to claim 3 for kinetic analysis of a compound,
wherein an excretion rate of the compound excreted from the apical face of the cell is determined based on the amounts B1 and B 1', the amounts B2 and B2', and the time interval t2.

5.  The method according to claim 3 for kinetic analysis of a compound,
wherein the method comprises:

an EA3 step, after the CE completion step, of feeding the third buffer solution to the third container, and adjusting and keeping an internal temperature of the third container to a fourth temperature lower than the third temperature, and
wherein the method further comprises:

measuring, in the EA3 step, amounts B3 and B3' of the compound in the third buffer solution at the time interval t2; and
determining an excretion rate of the compound excreted by passive diffusion from the basal/basolateral face of the cell based on the amounts B3 and B3' and the time interval t2.

6.  The method according to claim 5 for kinetic analysis of a compound,
wherein an excretion rate of the compound excreted via a transporter from the basal/basolateral face of the cell is determined based on the amounts B2 and B2', the amounts B3 and B3', and the time interval t2.

7.  The method according to claim 1 for kinetic analysis of a compound,
wherein the method comprises:

conducting the CE step separately on a first container, a second container, and a third container each housing the cell and containing the compound;
a CE completion step of collecting the first buffer solution separately from the first container, the second container, and the third container; and
an EA2 step, after the CE completion step, of feeding a third buffer solution to the second container, and adjusting and keeping an internal temperature of the second container to a third temperature, and
wherein the method further comprises:

measuring amounts B2 and B2' of the compound in the third buffer solution at a time interval t2 in the EA2 step; and
determining an excretion rate of the compound excreted from the basal/basolateral face of the cell based on the amounts B2 and B2' and the time interval t2.

8.  The method according to claim 1 for kinetic analysis of a compound,
wherein the method comprises:

conducting the CE step separately on a first container, a second container, and a third container each housing the cell and containing the compound;
a CE completion step of collecting the first buffer solution separately from the first container, the second container, and the third container; and
an EA3 step, after the CE completion step, of feeding a third buffer solution to the third container, and adjusting and keeping an internal temperature of the third container to a fourth temperature lower than the third temperature, and
wherein the method further comprises:

measuring amounts B3 and B3' of the compound in the third buffer solution at a time interval t2 in the EA3 step; and

determining an excretion rate of the compound excreted by passive diffusion from the basal/basolateral face of the cell based on the amounts B3 and B3' and the time interval t2.

9. A method for kinetic analysis of a compound using a cell, the method comprising:

a CE step of feeding a first buffer solution separately to a first container, a second container, and a third container each containing a target compound and housing a cell, adjusting and keeping internal temperatures of the containers to a first temperature, and adjusting the internal temperatures of the containers to a second temperature lower than the first temperature;

a CE completion step of collecting the first buffer solution separately from the first container, the second container, and the third container; and

an EA1 step, after the CE completion step, of feeding a second buffer solution containing a reagent for removing cell-cell adhesion to the first container, and adjusting and keeping the internal temperature of the first container to a third temperature,

the method further comprising:

measuring amounts B1 and B1' of the compound in the second buffer solution at a time interval t2 in the EA1 step; and

determining an excretion rate of the compound excreted from a basal/basolateral face and an apical face of the cell based on the amounts B1 and B1' and the time interval t2.

10. A method for kinetic analysis of a compound using a cell, the method comprising:

a CE step of feeding a first buffer solution separately to a first container, a second container, and a third container each containing a target compound and housing a cell, adjusting and keeping internal temperatures of the containers to a first temperature, and adjusting the internal temperatures of the containers to a second temperature lower than the first temperature;

a CE completion step of collecting the first buffer solution separately from the first container, the second container, and the third container; and

an EA2 step, after the CE completion step, of feeding a third buffer solution to the second container, and adjusting and keeping the internal temperature of the second container to a third temperature,

the method further comprising:

measuring amounts B2 and B2' of the compound in the third buffer solution at a time interval t2 in the EA2 step; and

determining an excretion rate of the compound excreted from a basal/basolateral face of the cell based on the amounts B2 and B2' and the time interval t2.

11. A method for kinetic analysis of a compound using a cell, the method comprising:

a CE step of feeding a first buffer solution separately to a first container, a second container, and a third container each containing a target compound and housing a cell, adjusting and keeping internal temperatures of the containers to a first temperature, and adjusting the internal temperatures of the containers to a second temperature lower than the first temperature;

a CE completion step of collecting the first buffer solution separately from the first container, the second container, and the third container; and

an EA3 step, after the CE completion step, of feeding a third buffer solution to the third container, and adjusting and keeping the internal temperature of the third container to a fourth temperature lower than the third temperature,

the method further comprising:

measuring amounts B3 and B3' of the compound in the third buffer solution at a time interval t2 in the EA3 step; and

determining an excretion rate of the compound excreted by passive diffusion from a basal/basolateral face of the cell based on the amounts B3 and B3' and the time interval t2.

12. The method according to any one of claims 1 to 11 for kinetic analysis of a compound,

wherein the method further comprises, before the CE step, an uptake step of exposing the cell to a solution containing the compound in a container or containers to allow the cell to take up the compound, and wherein the method further comprises:

measuring amounts A and A' of the compound in the solution at a time interval t3 in the uptake step; and determining an uptake rate of the compound by the cell based on the amounts A and A' and the time interval t3.

13. The method according to any one of claims 1 to 11 for kinetic analysis of a compound, wherein the cell comprises a hepatocyte.

# FIG.1

FIG.1

# FIG.2

| | STEP 0 | STEP 1 | STEP 2 | STEP 3 | STEP 4 | STEP 5 | STEP 6 |
|---|---|---|---|---|---|---|---|
| PLATE TEMPERATURE | 37℃ | 37℃ | 37℃→4℃ | 4℃ | 37℃→4℃ | (DIFFERENT DEPENDING ON TEST SECTION) | ROOM TEMPERATURE |
| [TEST SECTION 1] 37℃ DISRUPTED SYSTEM | CULTURE | WASHING CELLS TWICE WITH HANKS' SOLUTION, AND CONDITIONING WITH HANKS' SOLUTION FOR 10 MIN., FOLLOWED BY REMOVING HANKS' SOLUTION. | ADMINISTERING COMPOUND, AND, 30 MIN. LATER, COOLING PLATE TO 4℃ AND REMOVING COMPOUND. | WASHING THREE TIMES WITH ICE-COLD HANKS' SOLUTION. | FEEDING HANKS' SOLUTION, AND, 30 MIN. LATER, COOLING PLATE TO 4℃, AND COLLECTING SUPERNATANT. | FEEDING HANKS' SOLUTION CONTAINING EGTA AND, 30 MIN. LATER, COLLECTING SUPERNATANT. | (FLUORESCENCE MEASUREMENT) FEEDING 1% TRITON X-100 SOLUTION AND COLLECTING TOTALITY OF CELL LYSATE. |
| [TEST SECTION 2] 37℃ MAINTAINED SYSTEM | | | | | | FEEDING HANKS' SOLUTION AND, 30 MIN. LATER, COLLECTING SUPERNATANT. | (LMS ANALYSIS) FEEDING METHANOL AND COLLECTING TOTALITY OF CELL LYSATE. |
| [TEST SECTION 3] 4℃ MAINTAINED SYSTEM | | | | | | FEEDING HANKS' SOLUTION AND, 30 MIN. LATER, COLLECTING SUPERNATANT. | |

EP 3 933 045 A1

## FIG.3

| | STEP 4 | STEP 5 | STEP 6 | IMAGE VIEW |
|---|---|---|---|---|
| [TEST SECTION 1] 37°C DISRUPTED SYSTEM | FIRST BASOLATERAL EFFLUX DIFFUSION (1)' + TP (2)' (S1) | BILIARY EXCRETION (3) + SECOND BASOLATERAL EFFLUX DIFFUSION (1)' + TP (2)' (B1) | REMAINING IN CELL (4) (C1) | (C1) |
| [TEST SECTION 2] 37°C MAINTAINED SYSTEM | FIRST BASOLATERAL EFFLUX DIFFUSION (1)' + TP (2)' (S2) | SECOND BASOLATERAL EFFLUX DIFFUSION (1)' + TP (2)' (B2) | REMAINING IN CELL (4) + BILIARY EXCRETION (3) (C2) | (C2) |
| [TEST SECTION 3] 4°C MAINTAINED SYSTEM | FIRST BASOLATERAL EFFLUX DIFFUSION (1)' + TP (2)' (S3) | SECOND BASOLATERAL EFFLUX DIFFUSION (1) (B3) | REMAINING IN CELL (4) + BILIARY EXCRETION (3) (C3) | (C3) |

# FIG.4

| STEP | DEFINITION | PATTERN 1 | PATTERN 2 | FRACTION | CORRESPONDING TO |
|---|---|---|---|---|---|
| 4 | FRACTION OF FIRST BASOLATERAL EFFLUX (1)'+(2)' | | 70.82 | Sup | S1 |
| 5 | FRACTIONS OF BILIARY EXCRETION AND SECOND BASOLATERAL EFFLUX (1)+(2)+(3) | | 24.32 | | B1 |
| 5 | FRACTION OF SECOND BASOLATERAL EFFLUX (1)+(2) | | 18.90 | | B2 |
| 5 | FRACTION OF SECOND BASOLATERAL EFFLUX (ONLY BY DIFFUSION) (1) | 38.15 | 11.13 | ExEfx-Dif | B3 |
| 5 | FRACTION OF SECOND BASOLATERAL EFFLUX (ONLY VIA TRANSPORTER) (2) | 26.60 | 7.76 | ExEfx-TP | B2-B3 |
| 5 | FRACTION OF BILIARY EFFLUX (3) | 18.58 | 5.42 | BCEfx | B1-B2 |
| 6 | FRACTION REMAINING IN CELL (4) | 16.67 | 4.86 | Cell | C1 |
| 6 | FRACTION OF BILIARY EXCRETION AND FRACTION REMAINING IN CELL (3)+(4) | 30.88 | 9.01 | | C2 |
| TOTAL | | 100.00 | 100.00 | | |

# FIG.5

PATTERN 1 : 10 μM CDF

PATTERN 2 : 10 μM CDF

# FIG.6

PATTERN 1 : 10 μM RHODAMINE 123

ExEfx-Dif
10.95%

ExEfx-TP
12.11%

BCEfx
4.92%

Cell
72.02%

▓ExEfx-Dif
▓ExEfx-TP
▣BCEfx
▢Cell

PATTERN 2 : 10 μM RHODAMINE 123

Cell
43.72%

Sup
39.29%

ExEfx-Dif
6.65%

ExEfx-TP
7.35%

BCEfx
2.99%

▓Sup
▓ExEfx-Dif
▓ExEfx-TP
▣BCEfx
▢Cell

# FIG.7

# FIG.8

| 106 | 107 | 108 | 109 |
|---|---|---|---|
| | 107B | 108A | |
| | 107C | 108B | |

107A

# FIG.9

204
205

206

201
202
203

207 208 209 210 211 212 213

214

FIG.10

# FIG.11(a)

| STEP NUMBER | SUBSTEP NUMBER | OPERATION | PLATE TEMPERATURE |
|---|---|---|---|
| 0 | 1 | CULTURE OF CELL | 37°C |
| - | 2 | TRANSFERRING CULTURE PLATE CONTAINING CELL FROM CULTURE UNIT TO SAMPLE PREPARATION UNIT AND SETTING THE SAME TO SAMPLE PREPARATION UNIT | 37°C |
| 1 | 3 | REMOVING MEDIUM FROM SET CULTURE PLATE | 37°C |
| 1 | 4 | ASPIRATING BUFFER FROM DRUG SOLUTION RACK | 37°C |
| 1 | 5 | FEEDING BUFFER TO WELLS IN CULTURE PLATE | 37°C |
| 1 | 6 | REMOVING BUFFER FROM WELLS IN CULTURE PLATE | 37°C |
| 1 | 7 | REPEATING SUBSTEPS 4 TO 6 TWICE IN TOTAL | 37°C |
| 1 | 8 | ASPIRATING BUFFER FROM DRUG SOLUTION RACK | 37°C |
| 1 | 9 | FEEDING BUFFER TO CULTURE PLATE AND CONDITIONING FOR 10 MIN. | 37°C |
| 1 | 10 | REMOVING BUFFER FROM WELLS IN CULTURE PLATE | 37°C |
| 2 | 11 | ASPIRATING DRUG SOLUTION FROM DRUG SOLUTION RACK | 37°C |
| 2 | 12 | FEEDING DRUG SOLUTION TO CULTURE PLATE AND INCUBATING FOR 30 MIN. | 37°C |
| 2 | 13 | COOLING CULTURE PLATE HOLDER FROM 37°C TO 4°C | 37°C→4°C |
| 2 | 14 | REMOVING DRUG SOLUTION FROM WELLS IN CULTURE PLATE | 4°C |
| 3 | 15 | ASPIRATING BUFFER ADJUSTED TO 4°C FROM DRUG SOLUTION RACK | 4°C |
| 3 | 16 | FEEDING BUFFER TO WELLS IN CULTURE PLATE | 4°C |
| 3 | 17 | REMOVING BUFFER FROM WELLS IN CULTURE PLATE | 4°C |
| 3 | 18 | REPEATING SUBSTEPS 13 TO 15 THREE TIMES IN TOTAL | 4°C |
| - | 19 | WARMING CULTURE PLATE HOLDER FROM 4°C TO 37°C | 4°C→37°C |
| 4 | 20 | ASPIRATING BUFFER FROM DRUG SOLUTION RACK | 37°C |
| 4 | 21 | FEEDING BUFFER TO CULTURE PLATE AND INCUBATING FOR 30 MIN. | 37°C |
| 4 | 22 | COOLING CULTURE PLATE HOLDER FROM 37°C TO 4°C | 37°C→4°C |
| 4 | 23 | COLLECTING SUPERNATANT FROM WELLS IN CULTURE PLATE TO ANOTHER MULTI-WELL PLATE | 4°C |
| - | 24 | WARMING CULTURE PLATE HOLDER FROM 4°C TO 37°C | 4°C→37°C |

# FIG.11(b)

| STEP NUMBER | SUBSTEP NUMBER | OPERATION | PLATE TEMPERATURE |
|---|---|---|---|
| 5 | 25 | [WELL FOR TEST SECTION 1]<br>ASPIRATING BUFFER CONTAINING EGTA FROM DRUG SOLUTION RACK | 37°C |
| 5 | 26 | [WELL FOR TEST SECTION 1]<br>FEEDING BUFFER CONTAINING EGTA TO CULTURE PLATE AND INCUBATING FOR 30 MIN. | 37°C |
| 5 | 27 | [WELL FOR TEST SECTION 2]<br>ASPIRATING BUFFER FROM DRUG SOLUTION RACK | 37°C |
| 5 | 28 | [WELL FOR TEST SECTION 2]<br>FEEDING BUFFER TO CULTURE PLATE AND INCUBATING FOR 30 MIN. | 37°C |
| 5 | 29 | [WELL FOR TEST SECTION 3]<br>ASPIRATING BUFFER FROM DRUG SOLUTION RACK | 4°C |
| 5 | 30 | [WELL FOR TEST SECTION 3]<br>FEEDING BUFFER TO CULTURE PLATE AND INCUBATING FOR 30 MIN. | 4°C |
| 5 | 31 | [WELL FOR TEST SECTION 1]<br>COLLECTING SUPERNATANT FROM WELL FOR TEST SECTIONS 1 TO ANOTHER MULTI-WELL PLATE | 37°C |
| 5 | 32 | [WELL FOR TEST SECTION 2]<br>COLLECTING SUPERNATANT FROM WELL FOR TEST SECTION 2 TO ANOTHER MULTI-WELL PLATE | 37°C |
| 5 | 33 | [WELL FOR TEST SECTION 3]<br>COLLECTING SUPERNATANT FROM WELL FOR TEST SECTION 3 TO ANOTHER MULTI-WELL PLATE | 4°C |
| - | 34 | CHANGING TEMPERATURE OF CULTURE PLATE FROM 37°C/4°C TO ROOM TEMPERATURE | 37°C/4°C→ ROOM TEMPERATURE |
| 6 | 35 | ASPIRATING 1% Triton X-100 OR PURE WATER/METHANOL FROM DRUG SOLUTION RACK | ROOM TEMPERATURE |
| 6 | 36 | FEEDING 1% Triton X-100 OR PURE WATER/METHANOL | ROOM TEMPERATURE |
| 6 | 37 | COLLECTING TOTALITY OF CELL SUSPENSION | ROOM TEMPERATURE |
| - | 38 | TRANSFERRING PLATES INCLUDING COLLECTED DRUG SOLUTION TO MEASUREMENT UNIT | - |
| - | 39 | MEASUREMENT WITH PLATE READER OR LCMS | - |
| - | 40 | DETERMINATION OF DISTRIBUTION RATIO AND SCORES OF FRACTIONS, AND EXCRETION RATES AND UPTAKE RATES FROM MEASUREMENT RESULTS | - |
| - | 41 | DISPLAYING DETERMINED VALUES ON DISPLAY UNIT | - |

## INTERNATIONAL SEARCH REPORT

| International application No. |
| --- |
| PCT/JP2019/007245 |

### A. CLASSIFICATION OF SUBJECT MATTER
Int. Cl. C12Q1/02(2006.01)i

According to International Patent Classification (IPC) or to both national classification and IPC

### B. FIELDS SEARCHED

Minimum documentation searched (classification system followed by classification symbols)
Int. Cl. C12Q1/02

Documentation searched other than minimum documentation to the extent that such documents are included in the fields searched

| | |
| --- | --- |
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580 (JDreamIII), CAplus/MEDLINE/EMBASE/BIOSIS/WPIDS (STN)

### C. DOCUMENTS CONSIDERED TO BE RELEVANT

| Category* | Citation of document, with indication, where appropriate, of the relevant passages | Relevant to claim No. |
| --- | --- | --- |
| Y | WO 2016/166995 A1 (HITACHI HIGH-TECHNOLOGIES CORPORATION) 20 October 2016, paragraphs [0010], [0026]-[0073], fig. 2-4 & JP 2016-202024 A & US 2018/0120296 A6, fig. 2-4 & EP 3284813 A1 & CN 107406814 A | 1-13 |
| Y | WO 2015/080106 A1 (HITACHI HIGH-TECHNOLOGIES CORPORATION) 04 June 2015, paragraphs [0009], [0020]-[0037], fig. 2, 3 & JP 2015-105842 A & US 2016/0209403 A6, fig. 2, 3 & US 2018/0106783 A & EP 3023489 A1 & EP 3272852 A1 & CN 105378053 A & CN 107513550 A & CN 107677779 A | 1-13 |

| ☒ Further documents are listed in the continuation of Box C. | ☐ See patent family annex. |
| --- | --- |

| * Special categories of cited documents: | | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 12.04.2019 | 07.05.2019 |

| Name and mailing address of the ISA/ | Authorized officer |
| --- | --- |
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

| International application No. |
|---|
| PCT/JP2019/007245 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2003-528889 A (CITY OF HOPE) 30 September 2003, paragraph [0067] & WO 2001/072837 A2, example 5 | 1-13 |
| Y | CN 102440848 A (TIANJIN PHARMACEUTICAL RESEARCH INSTITUTE) 09 May 2012, paragraph [0025], table 2 (Family: none) | 1-13 |
| Y | LIU X. et al., Partial Maintenance of Taurocholate Uptake by Adult Rat Hepatocytes Cultured in a Collagen Sandwich Configuration, Pharmaceutical Research, 1998, vol. 15, no. 10, pp. 1533-1539, fig. 3, 4 | 12 |

Form PCT/ISA/210 (continuation of second sheet) (January 2015)

**REFERENCES CITED IN THE DESCRIPTION**

**Patent documents cited in the description**

- WO 2005118787 A
- WO 9412662 A
- WO 2015080106 A
- WO 2016166995 A